# FASCICULE DE BREVET EUROPEEN

(11) **EP 0 053 071 B1**
(45) Date de publication et mention de la délivrance du brevet: **10.10.1984**
(21) Numéro de dépôt: 81401828.9
(22) Date de dépôt: 19.11.1981
(51) Int. Cl.: C07D 498/04, A61K 31/535

(54) **Nouvelles oxacéphalosporines, leur préparation et les médicaments qui les contiennent**
Oxacephalosporine, ihre Herstellung und sie enthaltende Arzneimittel
Oxacephalosporins, their preparation and therapeutic agents containing them

(30) Priorité: 20.11.1980 FR 8024639
(43) Date de publication de la demande: 02.06.1982
(73) Titulaire: RHONE-POULENC SANTE, 92400 Courbevoie Cédex (FR)
(72) Inventeur: Farge, Daniel, F-94320 Thiais (FR); Le Roy, Pierre, F-94320 Thiais (FR); Moutonnier, Claude, F-92350 Le Plessis-Robinson (FR); Plau, Bernard, F-94000 Créteil (FR); Peyronel, Jean-François, F-91110 Palaiseau (FR)
(74) Mandataire: Gaumont, Robert

## Description

La présenté invention concerne de nouvelles oxacéphalosporines de formule générale: 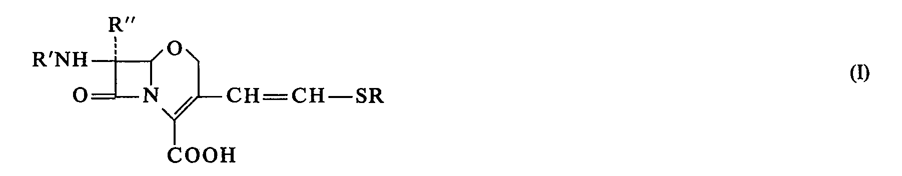 leurs sels, leur préparation et les médicaments qui les contiennent.

Dans la demande française 2 385 722 ont été décrites les céphalosporines et oxacéphalosporines de formule générale: 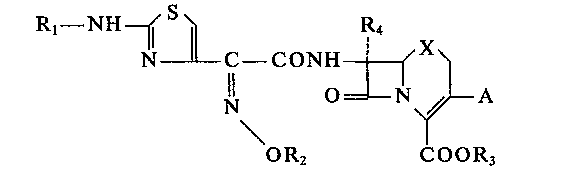 dans laquelle A est notamment un radical alcényloxy ou un radical hétérocyclylthiométhyle. Ces produits présentent des propriétés antibactériennes.

Dans la demande française 2 361 399 ont été décrites des oxacéphalosporines de formule générale: 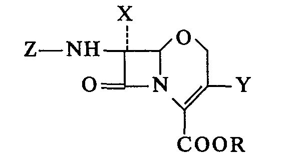 dans laquelle Z représente notamment un radical 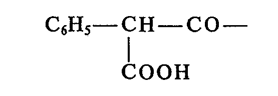 et Y est entre autres un radical hétérocyclylthio. Ces produits sont également des agents antibactériens.

Dans la demande française 2 137 899 ont été décrites les céphalosporines de formule générale: 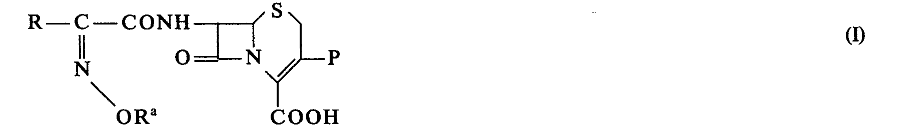 dans laquelle P représente notamment des radicaux alcoyloxyvinyle ou cyanovinyle. Ces céphalosporines sont des agents antibactériens.

Dans la formule générale (I), le symbole R est choisi parmi les significations:
1) dioxo-5,6 tétrahydro-1,4,5,6 triazine-1,2,4 yle-3 substitué en position -4 par
   a) un radical alcoyle contenant 1 ou 2 atomes de carbone éventuellement substitué par un radical alcoyloxy, alcoylthio, formyle,
   b) un radical allyle, dihydroxy-2,3 propyle, dihydroxy-1,3 propyle-2 ou formyl-2 hydroxy-2 éthyle,
   c) un radical alcoyle contenant 2 ou 3 atomes de carbone substitué par hydroxy, carbamoyloxy, alcanoyloxy ou alcanoylamino (dont les portions alcanoyle sont non substituées ou substituées par amino), alcoylsulfonylamino, uréido, alcoyluréido ou dialcoyluréido,
2) dioxo-5,6 tétrahydro-1,4,5,6 triazine-1,2,4 yle-3 substitué en position -1 par un radical alcoyle contenant 1 ou 2 atomes de carbone éventuellement substitué par un radical formyle,
3) thiadiazol-1,3,4 yle-5 substitué par alcoyle, dialcoylaminoalcoyle ou alcanoylaminoalcoyle,

le symbole R' représente un radical de formule générale: 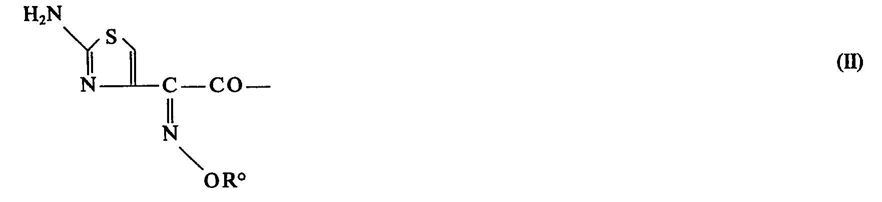 [dans laquelle R^{O} est un atome d'hydrogène, un radical alcoyle, vinyle, ou carboxyalcoyle représenté par la formule générale: 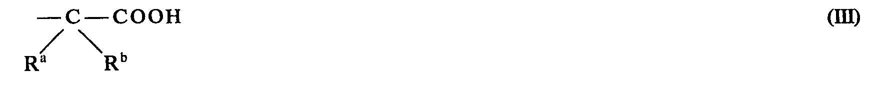 dans laquelle les radicaux R" et R^{b}, qui sont identiques ou différents, représentent des atomes d'hydrogène ou des radicaux alcoyle, ou forment ensemble un radical alcoylène contenant 2 ou 3 atomes de carbone], ou R' représente un radical a-carboxyarylacétyle dans lequel aryle est un radical phényle (éventuellement substitué par un radical p.hydroxy) ou un radical thiényle-2 ou -3, et
le symbole R" représente un atome d'hydrogène ou un radical méthoxy en position 7a.

Il est entendu que les portions ou radicaux alcoyles ou alcanoyles cités ci-dessus (ou qui seront cités ci-après) sont (sauf mention spéciale) droits ou ramifiés et contiennent 1 à 4 atomes de carbone.

Il est également entendu que le substituant en position -3 des produits de formule générale (I) peut se présenter sous forme cis ou trans ou d'un mélange des formes cis et trans.

Dans ce qui suit la stéréoisomérie trans sera désignée par E et la stéréoisomérie cis sera désignée par Z.

Par ailleurs, il est entendu que le groupement OR° du radical de formule générale (II) peut se trouver dans l'une des positions syn ou anti et que ces isomères et leurs mélanges entrent dans le cadre de la présente invention.

La forme syn peut être représentée par la formule:

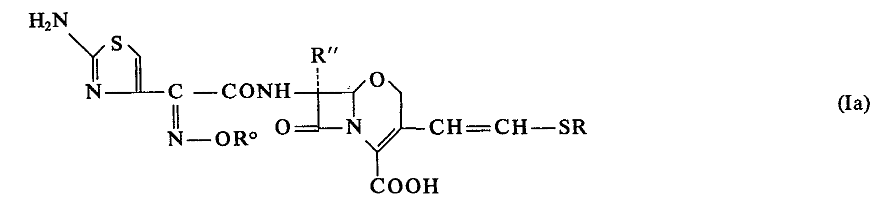

La forme anti peut être représentée par la formule:

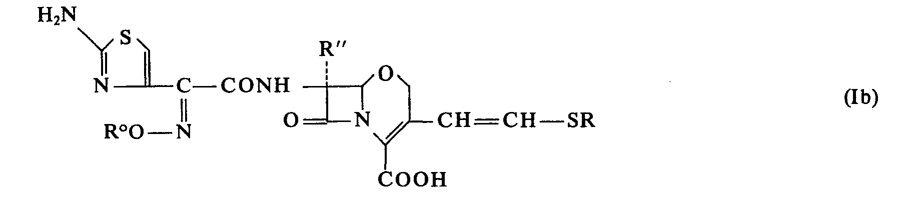

Lorsque le radical R est un radical tétrahydro-1,4,5,6 triazinyle substitué en position -1 ou -4 ou tétrahydro-1,2,5,6 triazinyle substitué en position -2, il peut être représenté par les formes tautomères:

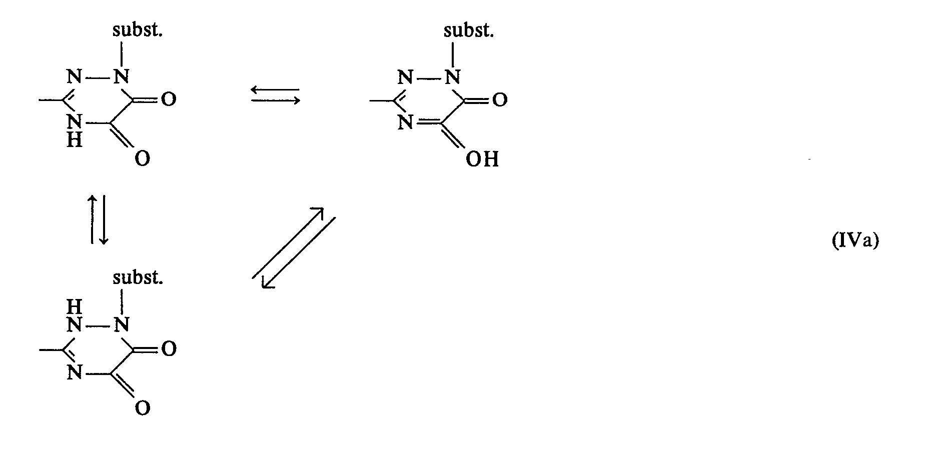

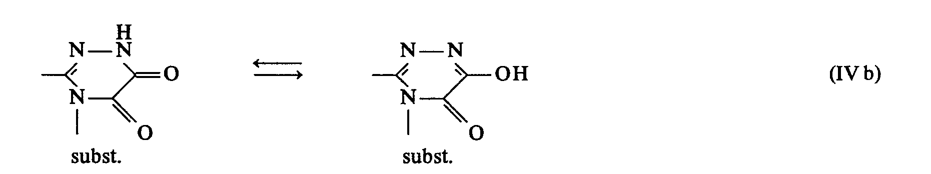

Lorsque le radical R contient un substituant formylalcoyle, il peut se présenter sous sa forme aldéhyde libre ou hydrate d'aldéhyde. On observe notamment ces formes dans les conditions décrites ci-dessous. Les études de résonance magnétique nucléaire montrent en particulier que lorsque R est dioxo-5,6 formylméthyl-4 tétrahydro-1,4,5,6 triazine-1,2,4 yle-3:
- en solvant acide, tel que l'acide formique ou trifluoroacétique (deutérés), en présence ou non d'eau (lourde) le produit se présente principalement sous la forme d'aldéhyde libre.
- en solvant basique tel que l'eau (lourde) additionnée de bicarbonate de sodium, il se présente principalement sous la forme d'hydrate d'aldéhyde.
- en solvant neutre tel que diméthylsulfoxyde (d₆), les formes aldéhyde libre et hydrate d'aldéhyde sont présentes, l'addition d'eau déplaçant l'équilibre en faveur de la forme hydrate d'aldéhyde.

En général on préfère les produits de formule générale (I a).

Parmi les significations du symbole R ci-dessus, on peut citer notamment:
méthyl-2 thiadiazol-1,3,4 yle-5
éthyl-2 thiadiazot-1,3,4 yle-5
diméthylaminométhyl-2 thiadiazol-1,3,4 yle-5
[diméthylamino-2 éthyl]-2 thiadiazol-1,3,4 yle-5
acétamidométhyl-2 thiadiazol-1,3,4 yle-5
(acétamido-2 éthyl)-2 thiadiazol-1,3,4 yle-5
dioxo-5,6 méthyl-4 tétrahydro-1,4,5,6 triazine-1,2,4 yle-3
dioxo-5,6 éthyl-4 tétrahydro-1,4,5,6 triazine-1,2,4 yle-3
allyl-4 dioxo-5,6 tétrahydro-1,4,5,6 triazine-1,2,4 yle-3
dioxo-5,6 (hydroxy-2 éthyl)-4 tétrahydro-1,4,5,6 triazine-1,2,4 yle-3
dioxo-5,6 méthoxyméthyl-4 tétrahydro-1,4,5,6 triazine-1,2,4 yle-3
dioxo-5,6 (méthoxy-2 éthyl)-4 tétrahydro-1,4,5,6 triazine-1,2,4 yle-3
(acétamido-2 éthyl)-4 dioxo-5,6 tétrahydro-1,4,5,6 triazine-1,2,4 yle-3
dioxo-5,6 méthylthiométhyl-4 tétrahydro-1,4,5,6 triazine-1,2,4 yle-3
dioxo-5,6(méthylthio-2 éthyl)-4 tétrahydro-1,4,5,6 triazine-1,2,4 yle-3
(carbamoyloxy-2 éthyl)-4 dioxo-5,6 tétrahydro-1,4,5,6 triazine-1,2,4 yle-3
dioxo-5,6 (formyloxy-2 éthyl)-4 tétrahydro-1,4,5,6 triazine-1,2,4 yle-3
(acétoxy-2 éthyl)-4 dioxo-5,6 tétrahydro-1,4,5,6 triazine-1,2,4 yle-3
dioxo-5,6 (glycyloxy-2 éthyl)-4 tétrahydro-1,4,5,6 triazine-1,2,4 yle-3
(dihydroxy-2,3 propyl)-4 dioxo-5,6 tétrahydro-1,4,5,6 triazine-1,2,4 yle-3
(dihydroxy-1,3 propyl-2)-4 dioxo-5,6 tétrahydro-1,4,5,6 triazine-1,2,4 yle-3
dioxo-5,6 (formyl-2 hydroxy-2 éthyl)-4 tétrahydro-1,4,5,6 triazine-1,2,4 yle-3
dioxo-5,6 (méthylsulfonylamino-2 éthyl)-4 tétrahydro-1,4,5,6 triazine-1,2,4 yle-3
dioxo-5,6 (glycylamino-2 éthyl)-4 tétrahydro-1,4,5,6 triazine-1,2,4 yle-3
[(L) alanylamino-2 éthyl]-4 dioxo-5,6 tétrahydro-1,4,5,6 triazine-1,2,4 yle-3
dioxo-5,6 tétrahydro-1,4,5,6 (uréido-2 éthyl)-4 triazine-1,2,4 yle-3
dioxo-5,6 [(méthyl-3 uréido)-2 éthyl]-4 tétrahydro-1,4,5,6 triazine-1,2,4 yle-3
méthyl-1 dioxo-5,6 tétrahydro-1,4,5,6 triazine-1,2,4 yle-3
éthyl-1 dioxo-5,6 tétrahydro-1,4,5,6 triazine-1,2,4 yle-3
dioxo-5,6 formylméthyl-1 tétrahydro-1,4,5,6 triazine-1,2,4 yle-3
dioxo-5,6 formylméthyl-4 tétrahydro-1,4,5,6 triazine-1,2,4 yle-3
dioxo-5,6 (formyl-2 éthyl)-1 tétrahydro-1,4,5,6 triazine-1,2,4 yle-3
dioxo-5,6 (formyl-2 éthyl)-4 tétrahydro-1,4,5,6 triazine-1,2,4 yle-3

Lorsque R' représente un radical de formule générale (II), parmi les significations préférées du symbole R°, on peut citer notamment:
hydrogène, méthyle, propyle, isopropyle, butyle, isobutyle, sec.butyle, vinyle, carboxyméthyle ou carboxy-2 propyle.

A/- Selon l'invention, les produits de formule générale (I) peuvent être préparés par action d'un acide représenté par la formule générale: 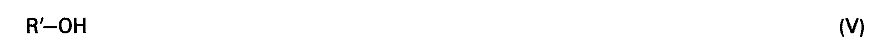 dans laquelle R' est défini comme précédemment (étant entendu que lorsque R' est un radical de formule générale (II), la fonction amine de ce radical est protégée) ou d'un dérivé réactif de cet acide, sur une amino-7 oxacéphalosporine de formule générale: 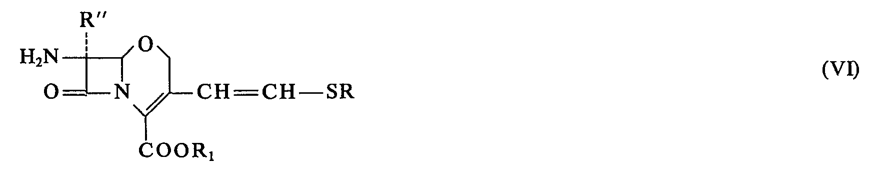 dans laquelle R et R" sont définis comme précédemment, R, représente un atome d'hydrogène, ou un radical protecteur d'acide facilement éliminable (par exemple méthoxyméthyle, t.butyle, benzhydryle, benzyle, nitrobenzyle ou p.méthoxybenzyle), suivie de l'élimination des radicaux protecteurs.
- Lorsque R' représente un radical de formule générale (II), l'acide de formule générale (V), sous forme syn ou anti ou leurs mélanges, conduit respectivement aux produits de formule générale (I) de forme syn ou anti ou leurs mélanges.
   II est entendu que l'oxime est protégé lorsque R^{o} représente un atome d'hydrogène.
   Lorsque R° contient un radical carboxy, celui-ci est également protégé.
- Lorsque R' représente un radical a-carboxyarylacétyle la protection du groupement carboxy n'est pas obligatoire; il peut donc être libre ou protégé.
   Il en est de même pour le radical hydroxy lorsque le groupement aryle représente p.hydroxyphényle.
- Lorsque R contient un substituant amino ou alcoylamino, ce groupement est protégé, et lorsqu'il contient un substituant hydroxy, ce dernier est libre ou de préférence protégé.

Il est entendu que les groupements amino, alcoylamino, carboxy et hydroxy qui existent dans certains radicaux sont (ou peuvent être) protégés par tous groupements protecteurs habituellement utilisés pour la protection des amines, des acides carboxyliques, des alcools ou des oximes et dont la mise en oeuvre n'altère pas le reste de la molécule.

### A titre d'exemples

- les groupements amino et alcoylamino sont protégés par des radicaux tels que t.butoxycarbonyle, trichloro-2,2,2 éthoxycarbonyle, trichloracétyle, trityle, benzyle, dibenzyle, benzyloxycarbonyle, p.nitrobenzyloxycarbonyle, p.méthoxybenzyloxycarbonyle, chloracétyle, formyle ou trifluoracétyle.
- les groupements carboxy sont ou peuvent être protégés par des radicaux tels que méthoxyméthyle, t.butyle, benzhydryle, benzyle, nitrobenzyle ou p.méthoxybenzyle.
- les groupements hydroxy, ou l'oxime de R' lorsque R^{O} est un atome d'hydrogène peuvent être (ou sont) protégés par des radicaux tels que trityle, tétrahydropyrannyle, méthoxy-2 propyle-2, alcoyloxycarbonyle (tel que t.butoxycarbonyle), aryloxycarbonyle (tel que benzyloxycarbonyle).

Les radicaux dihydroxy-2,3 propyle ou dihydroxy-1,3 propyle-2 peuvent être (ou sont) protégés sous forme d'un radical diméthyl-2,2 dioxolannyl-4 méthyle ou diméthyl-2,2 dioxannyle-5.

Lorsque l'on veut obtenir un produit de formule générale (I) dans laquelle R contient un radical formylalcoyle, ce radical peut être éventuellement protégé à l'état d'acétal, sous forme d'un radical de formule générale: 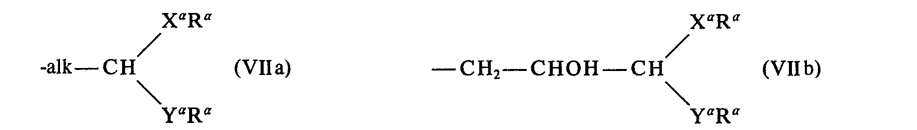 dans lesquelles alk est un radical alcoylène contenant 1 à 4 atomes de carbone, X" et Y" sont identiques et représentent des atomes d'oxygène ou de soufre et R" représente un radical alcoyle, ou bien X" et Y" sont identiques ou différents et représentent des atomes d'oxygène ou de soufre et les radicaux R" forment ensemble un radical alcoylène contenant 2 ou 3 atomes de carbone.

L'élimination du radical protecteur de R est effectuée avant, simultanément ou après l'élimination des autres radicaux protecteurs.

L'élimination des différents radicaux protecteurs peut s'effectuer simultanément ou successivement.

### A titre d'exemple

### 1 / L'élimination des groupements protecteurs d'amines s'effectue

- lorsqu'il s'agit d'un radical t.butoxycarbonyle, trityle, p.méthoxybenzyloxycarbonyle ou formyle: par traitement en milieu acide. On utilise par exemple l'acide trifluoracétique en opérant à une température comprise entre 0 et 20°C, ou bien on utilise les acides formique, phosphorique ou polyphosphorique purs ou en présence d'eau à une température comprise entre 20 et 60°C ou encore l'acide paratoluènesulfonique ou méthanesulfonique dans l'acétone ou l'acétonitrile, à une température comprise entre 20°C et la température de reflux du mélange réactionnel. Dans ces conditions lorsque R' est un radical de formule générale (II) le produit de formule générale (I) peut être obtenu sous forme de trifluoracétate, de solvate avec l'acide formique, de phosphate, de méthanesulfonate ou de paratoluènesulfonate, dont on peut libérer la fonction amine par toute méthode connue en soi pour obtenir une amine à partir de l'un de ses sels sans toucher au reste de la molécule. On opère notamment par mise en contact avec une résine échangeuse d'ions ou par action d'une base organique;
- lorsqu'il s'agit d'un radical trichloro-2,2,2 éthoxycarbonyle ou p.nitrobenzyloxycarbonyle: par réduction (notamment traitement par le zinc dans l'acide acétique);
- lorsqu'il s'agit d'un radical chloracétyle ou trichloracétyle: par application de la méthode décrite dans le brevet français publié sous le n° 2 243 199;
- lorsqu'il s'agit d'un radical benzyle, dibenzyle ou benzyloxycarbonyle: par hydrogénation catalytique;
- lorsqu'il s'agit d'un radical trifluoracétyle: par traitement en milieu basique.

### 2/ L'élimination des groupements protecteurs du radical carboxy s'effectue

- lorsqu'il s'agit d'un groupement t.butyle, p.méthoxybenzyle ou benzhydryle: par traitement en milieu acide, dans les conditions décrites ci-dessus pour l'élimination du radical trityle protecteur d'amino. Dans le cas du radical benzhydryle, on peut opérer en présence d'anisole;
- lorsqu'il s'agit d'un groupement méthoxyméthyle: par traitement en milieu acide dilué;
- lorsqu'il s'agit d'un groupement nitrobenzyle: par réduction (notamment traitement par le zinc dans l'acide acétique ou hydrogénolyse).

### 3/L'élimination des groupements protecteurs de l'oxime et/ou des radicaux hydroxy s'effectue

- lorsqu'il s'agit de groupement trityle ou tétrahydropyrannyle, ou des radicaux diméthyl-2,2 dioxolannyl-4 méthyle ou diméthyl-2,2 dioxannyle-5: par acidolyse, par exemple par l'acide trifluoracétique, l'acide formique aqueux ou non, ou l'acide paratoluènesulfonique. Lorsqu'on utilise l'acide formique, aqueux ou non, la libération des radicaux hydroxy protégés à l'état d'acétal cyclique peut conduire au moins partiellement aux mono ou diesters correspondants, qui peuvent être séparés le cas échéant par chromatographie;
- lorsqu'il s'agit du groupement méthoxy-2 propyle-2: selon la méthode décrite dans le brevet belge 875379.
- lorsqu'il s'agit des groupements alcoyloxycarbonyle ou aryloxycarbonyle: selon les méthodes décrites dans le brevet belge 871 213.

### 4/ Le déblocage des groupements de formule générale (VII a ou VII b) (lorsque l'on veut obtenir un produit de formule générale (I) dans laquelle R contient un radical formylalcoyle), s'effectue:

- en présence d'un acide sulfonique (par exemple acide méthanesulfonique ou acide p.toluène- sulfonique) dans un solvant organique (par exemple acétonitrile ou acétone), éventuellement en présence d'eau et éventuellement en présence d'un réactif acétalisable tel que l'acétone, l'acide glyoxylique, le benzaldéhyde ou l'acide pyruvique, à une température comprise entre 20°C et la température de reflux du mélange réactionnel;
- ou bien, lorsque le radical R est un radical dioxo-5,6 tétrahydro-1,4,5,6 triazine-1,2,4 yle-3: par action d'acide formique aqueux (contenant de préférence moins de 10%d'eau), soit en présence ou non de silice, soit par transacétalisation en présence d'un réactif acétalisable tel que défini ci-dessus.
   a) Lorsque l'on utilise le produit de formule générale (V) sous forme d'acide, on effectue généralement la condensation de ce produit (dont le cas échéant les fonctions amine et/ou oxime ont été préalablement protégées), sur l'amino-7 oxacéphalosporine de formule générale (VI) dans laquelle, R étant défini comme précédemment, R₁ représente un radical protecteur facilement éliminable, en opérant dans un solvant organique tel que le diméthylformamide, l'acétonitrile, le tétrahydrofuranne, le dichlorométhane ou le chloroforme, en présence d'un agent de condensation tel qu'un carbodiimide (par exemple le dicyclohexylcarbodiimide), le NN'-carbonyldiimidazole ou l'éthoxy-2 éthoxycarbonyl-1 dihydro-1,2 quinoléine, à une température comprise entre -20 et 40°C puis on élimine les groupements protecteurs présents dans la molécule. Eventuellement, on opère en présence d'une quantité catalytique de N,N-diméthylamino-4 pyridine.
   b) Lorsque l'on utilise un dérivé réactif de l'acide de formule générale (V), il est possible de mettre en oeuvre l'anhydride, un anhydride mixte ou un ester réactif de formule générale: 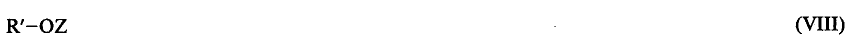 dans laquelle R' est défini comme ci-dessus et Z représente un radical succinimido, benzotriazolyle-1, nitro-4 phényle, dinitro-2,4 phényle, pentachlorophényle ou phtalimido. Lorsque R' est un radical de formule générale (II), la fonction amine de tels dérivés est préalablement protégée (par exemple comme décrit précédemment). Les conditions de protection des différents substituants sont telles que décrites précédemment.

Il est également possible de mettre en oeuvre des dérivés réactifs tels qu'un halogénure d'acide. Dans ce dernier cas, lorsque R' est un radical de formule générale (II) on peut, par exemple, faire réagir le chlorhydrate du chlorure d'acide sur l'amino-7 oxacéphalosporine de formule générale (VI) et, lorsque R' est un radical a-carboxy (p.hydroxyphényl) acétyle, le groupement hydroxy est libre ou protégé.

Lorsque l'on met en oeuvre l'anhydride, un anhydride mixte ou un halogénure d'acide (qui peuvent être préparés in situ), on effectue la condensation dans un solvant organique inerte tel qu'un éther (par exemple tétrahydrofuranne ou dioxanne), un solvant chloré (par exemple chloroforme ou chlorure de méthylène), un amide (par exemple diméthylformamide ou diméthylacétamide) ou une cétone (par exemple acétone), ou dans des mélanges de tels solvants en présence d'un accepteur d'acide tel qu'un époxyde (par exemple l'oxyde de propylène) ou tel qu'une base organique azotée comme la pyridine, la diméthylaminopyridine, la N-méthylmorpholine ou une trialcoylamine (par exemple triéthylamine), ou dans un milieu hydroorganique en présence d'un agent alcalin de condensation tel que le bicarbonate de sodium, et l'on opère à une température comprise entre -40 et +40°C, puis on remplace éventuellement les groupements protecteurs par des atomes d'hydrogène.

Lorsque l'on met en oeuvre un ester réactif de formule générale (VIII), on opère généralement en présence d'une trialcoylamine (par exemple triéthylamine) dans un solvant organique tel que le diméthylformamide à une température comprise entre 0 et 40°C, puis on remplace les groupements protecteurs par des atomes d'hydrogène.

B/- Selon l'invention, les produits de formule générale (1), peuvent également être préparés par action d'un thiol de formule générale: 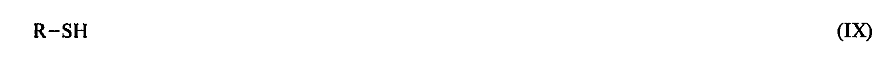 (ou d'un de ses sels alcalins ou alcalinoterreux) dans laquelle R, qui est défini comme ci-dessus, est protégé à l'état d'acétal [tel que défini par les formules générales (VII a) et (VII b)] lorsque l'on veut obtenir une oxacéphalosporine de formule générale (I) dans laquelle R contient un radical formyle, sur un dérivé d'oxacéphalosporine (ou le cas échéant sur un mélange des isomères de ce dérivé) de formule générale: 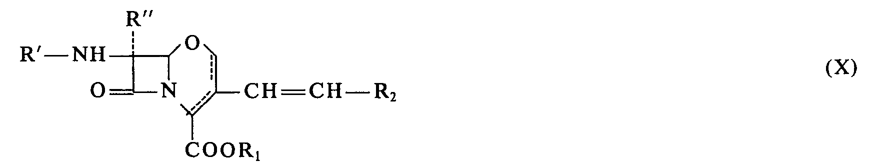 qui se présente sous forme bicyclooctène-2 ou -3 (selon la nomenclature des Chemical Abstracts), et dans laquelle R', R"et R₁ sont définis comme précédemment, le substituant sur l'atome de carbone en position -3 du bicyclooctène présente la stéréoisomérie E ou Z et R₂ représente un radical de formule générale: 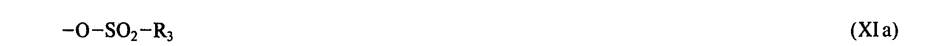 ou 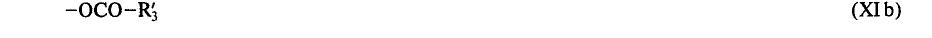 [dans lesquelles R₃ est un radical alcoyle, trifluorométhyle, trichlorométhyle ou un radical phényle éventuellement substitué par un atome d'halogène ou par un radical alcoyle ou nitro, et R3 est défini comme R₃ ou représente un radical alcanoylméthyle, alcanoyl-2 éthyle, alcanoyl-2 propyle, alcoyloxycarbonylméthyle, alcoyloxycarbonyl-2 éthyle, ou alcoyloxycarbonyl-2 propyle]
ou un atome d'halogène, suivie éventuellement de l'élimination des radicaux protecteurs.

Lorsque le radical R' représente un groupe de formule générale (II), le radical amino est libre ou protégé, lorsque R° représente un atome d'hydrogène, l'oxime est de préférence protégée et lorsque R° contient un groupement carboxy celui-ci est libre ou protégé.

Lorsque le radical R'représente un groupe a-carboxyarylacétyle, il est préférable de protéger le radical hydroxy lorsque le substituant aryle signifie p.hydroxyphényle; le groupement carboxy peut être libre ou protégé.

Lorsque le radical R du produit de formule générale (IX) est susceptible d'interférer avec la réaction, il est préférable de protéger ce groupement dans les conditions décrites précédemment (notamment lorsque R contient un radical amino, alcoylamino, hydroxy ou carboxy).

La protection et l'élimination de tous ces radicaux s'effectuent par exemple comme décrit précédemment.

Lorsque R₂ représente un atome d'halogène, il est choisi parmi le chlore, le brome et l'iode.

On opère généralement en présence d'une base organique, telle qu'une pyridine ou une base organique tertiaire du type: 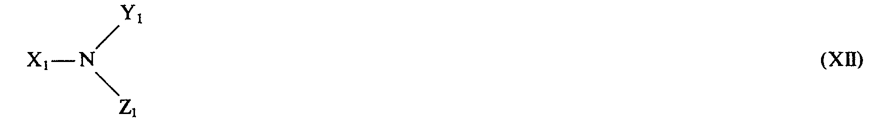 où X₁, Y₁ et Z₁ représentent des radicaux alcoyle ou phényle, ou éventuellement deux d'entre eux forment un cycle avec l'atome d'azote auquel ils sont rattachés. On utilise par exemple la diisopropyl- éthylamine ou la NN-diéthylaniline.

Lorsque l'on fait agir un sel alcalin ou alcalinoterreux du thiol de formule générale (IX), il n'est pas nécessaire d'opérer en présence d'une base organique telle que définie ci-dessus.

La réaction s'effectue avantageusement dans un solvant organique tel que le diméthylformamide, le diméthylacétamide, le méthanol, l'éthanol, le tétrahydrofuranne ou l'acétonitrile ou un mélange de tels solvants.

Il est également possible d'opérer en présence de bicarbonate alcalin dans un solvant tel que cité ci-dessus, éventuellement en présence d'eau.

On opère à une température comprise entre -20°C et la température de reflux du mélange réactionnel, la température choisie étant variable selon le thiol employé. De même, selon le thiol employé, la durée de réaction peut varier de 5 minutes à 48 heures.

Eventuellement on opère sous azote.

De préférence, lorsque l'on veut utiliser un bicyclooctène-3 de formule générale (X), on met en oeuvre un tel produit pour lequel R, est autre que l'hydrogène.

Les produits de formule générale (V) peuvent être préparés selon la méthode décrite dans le brevet belge 850 662, ou par application de la méthode décrite dans le brevet belge 877 884, lorsque R' est un radical de formule générale (II) dans laquelle R° est hydrogène ou alcoyle.

Les produits de formule générale (V) peuvent être préparés selon la méthode décrite dans le brevet belge 869 079, lorsque R' est un radical de formule générale (11) dans laquelle R' est vinyle.

Les produits de formule générale (V) peuvent être préparés selon les méthodes décrites dans les brevets belges 864 810,865 298, 876 541 et 876 542 lorsque R' est un radical de formule générale (II) dans laquelle R' est un substituant de formule générale (III).

Les produits de formule générale (V), lorsque R' est un radical a-carboxyarylacétyle, peuvent être préparés selon les méthodes suivantes:
- lorsque le groupement aryle représente un groupement p.hydroxyphényle: selon la méthode décrite dans la demande de brevet japonais 79 106 447 ou dans le brevet belge 852 912,
- lorsque le groupement aryle représente un groupement thiényle-2: selon D. Ivanov et N. Marekov: Compt. Rend. Acad. Bulgare Sci., 8(11), 29 (1955),
- Lorsque le groupement aryle représente un groupement thiényle-3: selon le brevet britannique 1125557.

Les amino-7 oxacéphalosporines de formule générale (VI) peuvent être obtenues selon l'un des schémas suivants:

A/ à partir d'un produit de formule générale: 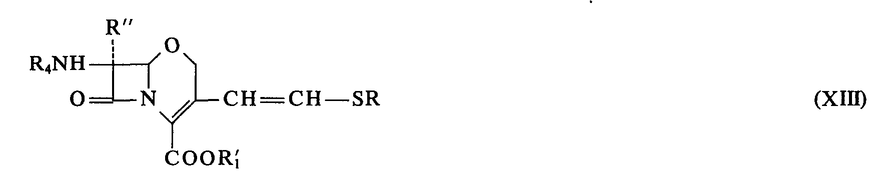 [dans laquelle R et R" sont définis comme précédemment, 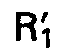 est défini comme R₁, à l'exeption de représenter un atome d'hydrogène, et R₄ représente un radical facilement éliminable], par élimination du radical R₄ (ou éventuellement élimination successive du radical R₄ et des autres radicaux ou groupements protecteurs).

Par radical R₄ facilement éliminable, on entend:
1 ) benzhydryle ou trityle
2) un radical acyle de formule générale: 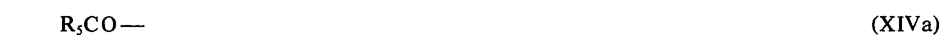 dans laquelle R₅ a les significants suivantes:
   a) hydrogène, alcoyle contenant 1 à 7 atomes de carbone, méthyle substitué par 1 à 3 atomes d'halogène, alcényle contenant 3 à 7 atomes de carbone ou cyanométhyle,
   b) un radical phényle pouvant être jusqu'à 3 fois substitué (par des atomes d'halogène ou par les radicaux hydroxy, nitro, cyano, triflourméthyle, alcoyle ou alcoyloxy) ou un radical thiényle-2 ou -3,
   c) un radical de formule générale: 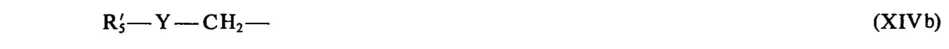 dans laquelle 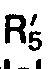 est un radical tel que défini en b) et Y est un atome de soufre ou d'oxygène,
   d) un radical arylalcoyle de formule générale: 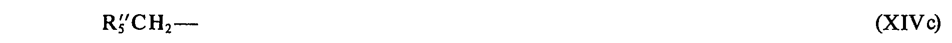 dans laquelle 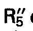 est un radical phényle pouvant être jusqu'à 3 fois. substitué (par des radicaux hydroxy, alcoyle ou alcoyloxy), ou un hétérocycle tel que thiényle-2 ou -3, furyle-2 ou -3, tétrazolyle-1,
3) un radical de formule générale: 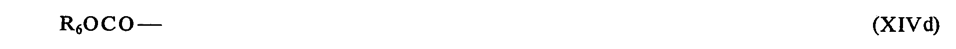 dans laquelle Rₐ est un radical alcoyle ramifié non substitué, un radical alcoyle droit ou ramifié portant un ou plusieurs substituants [tels que les atomes d'halogène ou les radicaux cyano, phényle, phényle substitué (par un ou plusieurs atomes d'halogène ou radicaux alcoyle, alcoyloxy, nitro ou phényle)], un radical triméthylsilyl-2 éthyle, un radical vinyle ou allyle, ou un radical quinolyle,
4) un radical de formule générale: 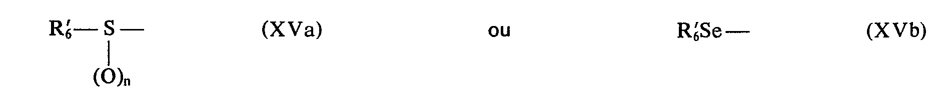 dans lesquelles le reste Ré est un radical alcoyle, phényle ou phényle substitué par un ou plusieurs atomes d'halogène ou radicaux nitro ou alcoyle et n est égal à 0 ou 1,
5) un radical bis(nitro-4 benzyl)phosphoryle,
6) ou bien R₄NH- peut être remplacé par un radical dialcoylamino-méthylèneamino ou par un radical de formule générale: 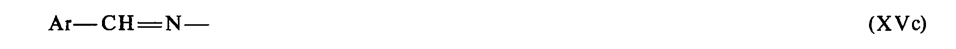 dans laquelle Ar est un groupe phényle éventuellement substitué par un ou plusieurs radicaux tels que alcoyle, alcoyloxy, hydroxy ou nitro.

Comme exemples de radicaux R₄ pouvant être utilisés, on peut citer les radicaux suivants:
formyle, acétyle, chloracétyle, trichloracétyle, phénylacétyle,
phénoxyacétyle, benzoyle,
t.butoxycarbonyle,
chloro-2 diméthyl-1,1 éthoxycarbonyle,
trichloro-2,2,2 éthoxycarbonyle,
trichloro-2,2,2 diméthyl-1,1 éthoxycarbonyle,
cyano-2 diméthyl-1,1 éthoxycarbonyle,
triméthylsilyl-2 éthoxycarbonyle,
benzyloxycarbonyle,
p.méthoxybenzyloxycarbonyle,
diméthoxy-3,5 benzyloxycarbonyle,
p.nitrobenzyloxycarbonyle,
diphénylméthoxycarbonyle,
(biphénylyl-4)-2 isopropyloxycarbonyle,
vinyloxycarbonyle,
allyloxycarbonyle,
quinolyl-8 oxycarbonyle,
o.nitrophénylthio,
p.nitrophénylthio,
bis(nitro-4 benzyl)phosphoryle.

Comme exemples de radicaux méthylèneamino définis précédemment en 6), on peut citer:
diméthylaminométhylèneamino,
diméthoxy-3,4 benzylidèneamino,
nitro-4 benzylidèneamino,
di t.butyl-3,5 hydroxy-4 benzylidèneamino.

L'élimination du radical protecteur R₄ s'effectue par toute méthode connue pour libérer une fonction amine sans toucher au reste de la molécule.

A titre d'exemple, on peut citer les méthodes suivantes:
- lorsque R₄ représente trityle, benzhydryle, trichloracétyle, chloracétyle, t.butoxycarbonyle, tri- chloréthoxycarbonyle, benzyloxycarbonyle, p.méthoxybenzyloxycarbonyle et p.nitrobenzyloxy- carbonyle: selon les méthodes citées ci-dessus pour la libération du radical amino du produit de formule générale (I);
- lorsque R₄ représente formyle, chloro-2 diméthyl-1,1 éthoxycarbonyle, cyano-2 diméthyl-1,1 éthoxycarbonyle, diméthoxy-3,5 benzyloxycarbonyle, diphénylméthoxycarbonyle, (biphénylyl-4)-2 isopropyloxycarbonyle, vinyloxycarbonyle, allyloxycarbonyle, quinolyl-8 oxycarbonyle, o.nitro- phénylthio, p.nitrophénylthio, et lorsque R₄NH- est remplacé par diméthylaminométhylèneamino, diméthoxy-3,4 benzylidèneamino ou nitro-4 benzylidèneamino: par acidolyse;
- lorsque R₄ représente di t.butyl-3,5 hydroxy-4 benzylidèneamino: par traitement par le réactif T de Girard, par analogie avec la méthode décrite dans le brevet belge 863 998;
- lorsquè R₄ représente trichloro-2,2,2 éthyle ou trichloro-2,2,2 diméthyl-1,1 éthoxycarbonyle: par traitement par le zinc dans l'acide acétique;
- lorsque R₄ représente acétyle, benzoyle, phénylacétyle ou phénoxyacétyle: selon la méthode décrite dans le brevet belge 758 800 ou selon la méthode décrite par Yoshioka, Tet. Letters 351 (1980);
- lorsque R₄ représente triméthylsilyléthoxycarbonyle: selon la méthode décrite par H. Gerlach, Helv. Chim. Acta 60(8), 3039 (1977);
- lorsque R₄ représente p.nitrobenzyloxycarbonyle ou benzyle: par hydrogénolyse en présence de palladium;
- lorsque R₄ représente bis(nitro-4 benzyl)phosphoryle: par application de la méthode décrite dans la demande de brevet japonais 77 125 185.

Lorsque R" est un groupe méthoxy, on pourra, de préférence aux méthodes précédentes, utiliser les méthodes suivantes:
1 ) Dans le cas où R₄ représente benzhydryle ou trityle: hydrogénolyse en présence de palladium.
2) Dans le cas où R₄ représente des radicaux de formules générale (XVa) ou (XVb) : par les méthodes décrites par E. M. Gordon, J. Am. Chem. Soc. 102(5), 1690 (1980); T. Kobayashi, Bull. Chem. Soc. Japan 52(11), 3366 (1979) et T. Kobayashi, Chem. Pharm. Bull. 27, 2718 (1979).
3) Dans le cas où R₄ représente un radical de formule générale (XVc): par acidolyse ou par traitement par le réactif T de Girard, par analogie avec la méthode décrite dans le brevet belge 863 998.

B/ à partir d'un produit de formule générale: 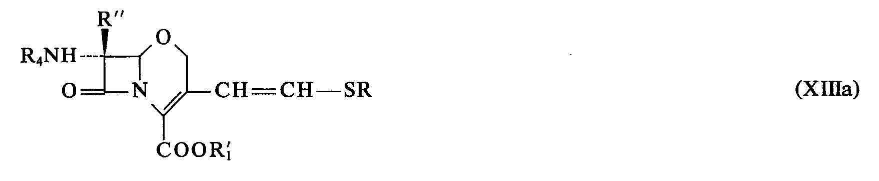 [dans laquelle R et Ri 'sont définis comme pour la formule générale (XIII), R" est un atome d'hydrogène en -7β et R₄ est un radical de formule générale (XIVa), (XVa), (XVb), (XVc) ou bis(nitro-4 benzyl)phosphoryle] par élimination du radical R₄ ou éventuellement élimination du radical R₄ et des radicaux protecteurs présents sur R, suivie de la transformation du produit obtenu en le produit méthoxylé correspondant en opérant dans les conditions décrites dans les brevets belges 871 213 ou 863 998 ou selon les méthodes décrites par T. Kobayashi et coll., Chem. Pharm. Bull., 27(11), 2718 (1979).

L'élimination du radical R₄ s'effectue dans les conditions décrites ci-dessus.

C/ à partir d'un produit de formule générale (Xllla) tel que défini ci-dessus, par méthoxylation dans les conditions citées ci-dessus, pour obtenir le produit correspondant de formule générale (XIII) dans laquelle R" est un radical méthoxy, puis élimination du radical R₄ comme décrit précédemment en A/.

D/ à partir d'un produit de formule générale (VI) dans laquelle R" est un atome d'hydrogène, par transformation en le dérivé méthoxylé correspondant de formule générale (VI), en opérant comme mentionné précédemment en C/.

Les produits de formule générale (XII et (Xllla) peuvent être obtenus par action d'un thiol de formule générale (IX) dont le radical R est éventuellement protégé, ou d'un de ses sels alcalins ou alcalinoterreux, sur un dérivé d'oxacéphalosporine ou le cas échéant sur un mélange d'isomères bicyclooctène-2 et -3 d'un dérivé de formule générale: 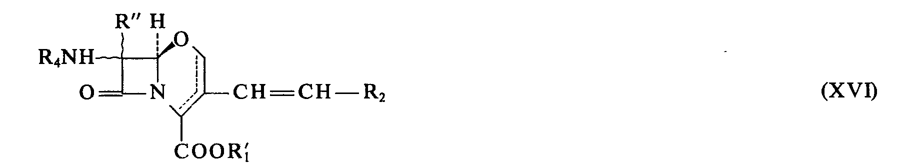 qui se présente sous forme bicyclooctène-2 ou -3, dans laquelle le substituant en position -3 du bicyclooctène présente la stéréoisomérie E ou Z et 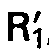 , R₂ et R₄ sont définis comme précédemment, étant entendu que lorsque l'on veut obtenir un produit de formule générale (XIII), le substituant R" est un atome d'hydrogène ou un radical méthoxy en 7a, et lorsque l'on veut obtenir un produit de formule générale (XIIIa) le substituant R" est un atome d'hydrogène en 7β.

La réaction s'effectue généralement dans les conditions décrites précédemment pour l'obtention d'une thiovinyl-3 oxacéphalosporine de formule générale (I) à partir d'un thiol de formule générale (IX) et d'un produit de formule générale (X).

Les thiols de formule générale (IX), qui peuvent être mis en oeuvre sous leur forme tautomère, peuvent être préparés par application d l'une des méthodes suivantes selon la signification du radical R:

### - lorsque R est

1° un radical dioxo-5,6 tétrahydro-1,4,5,6 triazine-1,2,4 yle-3 substitué en position -4 par un radical R^{γ} choisi parmi:
a) un radical allyle, alcoyle (1 ou 2 atomes de carbone), lui-même éventuellement substitué par un radical alcoyloxy ou alcoylthio,
b) un radical dihydroxy-2,3 propyle ou dihydroxy-1,3 propyle-2 (éventuellement protégé sous forme d'acétal cyclique),
c) un radical alcoyle (2 ou 3 atomes de carbone) lui-même substitué [par hydroxy, carbamoyloxy, dialcoylamino, alcoylsulfonylamino, alcanoylamino (éventuellement substitué), uréido, alcoyluréido ou dialcoyluréido],
d) un radical de formule générale (Vlla) ou (Vllb),

2° un radical dioxo-5,6 tétrahydro-1,4,5,6 triazine-1,2,4 yle-3 substitué en position -1 par un radical alcoyle contenant 1 ou 2 atomes de carbone ou par un radical de formule générale (VIIa): en faisant agir un oxalate d'alcoyle sur une thiosemicarbazide de formules générales: 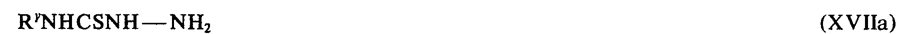 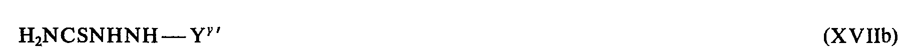 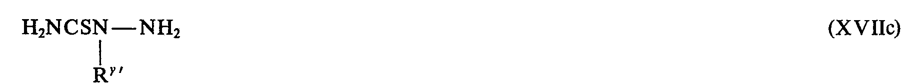 dans lesquelles R^{γ} a la définition donnée ci-dessus en 1° et R^{γ}' est un substituant défini ci-dessus en 2°, en présence d'un alcoolate alcalin, par exemple l'éthylate ou le méthylate de sodium, ou le t.butylate de potassium, par application de la méthode décrite par M. Pesson et M. Antoine, Bull. Soc. Chim. France 1590 (1970).

Il n'est pas absolument nécessaire de purifier le produit obtenu (ni de libérer les radicaux protégés) pour le mettre en oeuvre pour la préparation des produits de formule générale (I).

La thiosemicarbazide de formule générale (XVlla), (XVllb) ou (XVllc) peut préparée selon l'une des méthodes décrites par K. A. Jensen et coll., Acta Chem. Scand., 22, 1 (1968) ou par application de la méthode décrite par Y. Kazakov et J. Y. Potoskii, Doklady acad. Nauk. SSSR, 134, (1960), étant entendu que, lorsque R^{Y} contient un radical amino, ce dernier est protégé.

La protection du radical amino et l'élimination du radical protecteur s'effectuent selon les méthodes habituelles qui n'altèrent pas le reste de la molécule. On utilise notamment le groupement t.butoxy- carbonyle, qui peut être éliminé par hydrolyse acide.
- Lorsque R est un radical dioxo-5,6 tétrahydro-1,4,5,6 triazine-1,2;4 yle-3 substitué en position -4 par alcanoyloxyalcoyle (éventuellement substitué): par acylation respectivement de la dioxo-5,6 hydroxy-alcoyl-4 mercapto-3 tétrahydro-1,4,5,6 triazine-1,2,4 dont le radical mercapto a été préalablement protégé (par exemple selon C. G. Kruse et coll., Tet. Lett. 1725 (1976), par toute méthode connue pour acyler un alcool sans toucher au reste de la molécule, puis libération du groupement mercapto en milieu acide;
- Lorsque R est un radical thiadiazol-1,3,4 yle-5 éventuellement substitué par alcoyle: selon les méthodes décrites dans le brevet belge 830 821;
- Lorsque R est un radical thiadiazol-1,3,4 yle-5 substitué par dialcoylaminoalcoyle: selon la méthode décrite dans la demande de brevet allemand 2 446 254;
- Lorsque R est un radical thiadiazol-1,3,4 yle-5 substitué par un radical acylaminoalcoyle: selon la méthode dans la demande de brevet japonais 76 80857.

Les produits de formules générales (X) et (XVI) peuvent être préparés par action d'un dérivé activé des acides R₃S0₃H et R3COOH du type: 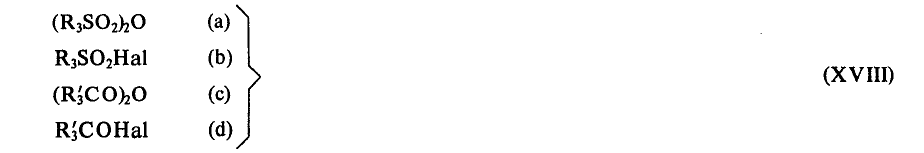 (R₃ et R3 étant définis comme précédemment et Hal étant un atome d'halogène), ou d'un d'halogénation, sur un produit de formule générale: 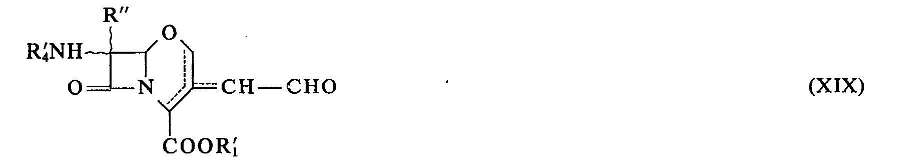 (ou un mélange de ses isomères) dans laquelle, 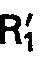 étant défini comme précédemment, le produit se présente sous forme bicyclooctène-2 ou -3, ou oxoéthylidène-3 bicyclooctane et
soit R4 est défini comme R' dans la formule générale (X) étant entendu que, lorsqu'il représente un radical de formule générale (II), l'amine de ce dernier est protégée
et R" est un atome d'hydrogène ou un radical méthoxy en position 7α, soit 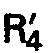 est défini comme R₄ dans la formule générale (XVI) et R" est un atome d'hydrogène ou un radical méthoxy en 7a, ou un atome d'hydrogène en 7β, suivie éventuellement de l'élimination des radicaux protecteurs.

Lorsque l'on désire mettre en oeuvre un produit de formule générale (XIX) dans laquelle R4 est un radical de formule générale (II) dont le reste R^{O} est un atome d'hydrogène, il est nécessaire de protéger préalablement l'oxime.

Lorsque l'on désire mettre en oeuvre un aldéhyde de formule générale (XIX) dans laquelle R4 contient un groupement carboxy, ce radical peut être libre ou protège si l'on fait un dérivé activé des acides R₃SO₃H ou 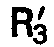 COOH; par contre il est nécessaire de le protéger préalablement si l'on fait agir un agent d'halogénation.

Lorsque l'on désire mettre en oeuvre un aldéhyde dans lequel le radical 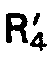 est a-carboxy (p.hydroxy- phényl)acétyle, il est nécessaire de protéger le radical hydroxy.

La protection et le cas échéant la libération des radicaux s'effectuent dans les conditions décrites précédemment.

On opère généralement en présence d'une base tertiaire telle que définie par la formule générale (XII), par exemple la triéthylamine ou la NN-diméthylaniline dans un solvant organique chloré (par exemple dichlorométhane), dans un ester (acétate d'éthyle), dans un éther (par exemple dioxanne, tétrahydrofuranne), dans un amide (par exemple diméthylacétamide, diméthylformamide) dans l'acétonitrile ou la N-méthylpyrrolidone ou dans un mélange de tels solvants, ou directement dans un solvant basique comme la pyridine ou bien, lorsque R₂ est autre qu'un atome d'halogène, on peut opérer en milieu hydroorganique en présence d'un agent alcalin de condensation (par exemple bicarbonate alcalin, soude ou potasse), à une température comprise entre -78°C et la température de reflux du mélange réactionnel.

Eventuellement on opère sous azote.

Il n'est pas absolument nécessaire d'avoir purifié l'intermédiaire de formule générale (XIX) pour mettre en oeuvre cette réaction.

Lorsque l'on veut préparer un produit de formule générale (X) ou (XVI) dans laquelle R₂ est un atome d'halogène, les agents d'halogénation peuvent être choisis parmi des dérivés halogénés du phosphore, notamment:
- les composés d'addition d'halogène et de triarylphosphite, ou bien
- le trichlorure de phosphore, l'oxychlorure de phosphore, le pentachlorure de phosphore, le di- chlorotriphénylphosphorane ou le catéchyltrichlorophosphorane lorsque R₂ est un atome de chlore ou
- le tribromure de phosphore, l'oxybromure de phosphore, le pentabromure de phosphore ou le caté- chyltribromophosphorane lorsque R₂ est un atome de brome.

Le catéchyltrichloro (ou tribromo) phosphorane, qui peut être préparé in situ, peut être obtenu selon la méthode décrite par H. Gross et U. Karsch, J. Prakt. Chem., 29, 315 (1965).

Les composés d'addition d'halogène et de triarylphosphite, qui peuvent être formés in situ, sont décrits par H. N. Rydon et B. L. Tonge, J. Chem. Soc., 3043 (1956), par J. Michalski et coll., J. Org. Chem., 45, 3122 (1980) ou dans le brevet belge 881424 et peuvent être préparés selon les méthodes mentionnées dans ces documents.

La préparation des dérivés halogénés de formule générale (X) ou (XVI) s'effectue en milieu anhydre.

Lorsque l'on veut préparer un produit de formule générale (X) ou (XVI), dans laquelle R₂ est un atome de chlore ou de brome, selon les conditions opératoires on peut isoler l'intermédiaire dihalogéné de formule générale: 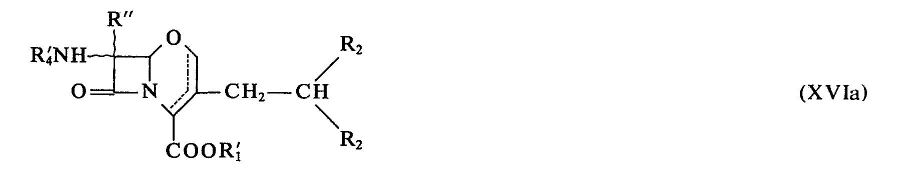 [dans laquelle R", R4, R; et R₂ étant définis comme ci-dessus, le produit présente la même isomérie que le produit de formule générale (X)] qui est ensuite déhydrohalogéné.

Lorsque l'on veut isoler l'intermédiaire dihalogéné, on opère par action d'un agent d'halogénation, dans un solvant organique tel qu'un solvant chloré (par exemple dichlorométhane, chloroforme, tétrachlorure de carbone, dichloro-1,2 éthane), un éther (par exemple éther éthylique, oxyde de propylène, tétrahydrofuranne, dioxanne), un amide (par exemple diméthylacétamide, diméthyl- propionamide, diméthylformamide, N-acétylmorpholine, N-acétylpipéridine, N-méthylpyrrolidone) ou un mélange de tels solvants, à une température un peu moins élevée que pour préparer le dérivé halogénovinyle correspondant, c'est-à-dire comprise entre -78 et 30°C.

I est également possible d'opérer en présence d'une base telle que la pyridine dans un solvant tel que cité ci-dessus, à une température comprise entre -78 et 0°C.

La déhydrohalogénation s'effectue en présence d'une base tertiaire telle que définie précédemment, d'une amine aromatique (par exemple pyridine, picoline, quinoléine), ou d'une base minérale (telle que la soude, la potasse, un carbonate ou un bicarbonate alcalin ou alcalino-terreux), en milieu organique ou hydroorganique dans des solvants tels que cités précédemment, à une température comprise entre - 20°C et la température de reflux du mélange réactionnel.

Il n'est pas absolutement nécessaire d'avoir purifié l' intermédiaire dihalogéné pour procéder à sa déhydrohalogénation.

Le cas échéant, l'élimination des radicaux protecteurs de la fonction amine et de la fonction acide s'effectue selon les méthodes décrites précédemment pour l'obtention du produit de formule générale (I).

Les produits de formule générale (X) peuvent également être obtenus par action d'un acide de formule générale (V) dont la fonction amine est préalablement protégée lorsque R' est un radical de formule générale (II), ou par action d'un de ses dérivés réactifs, sur un produit de formule générale: 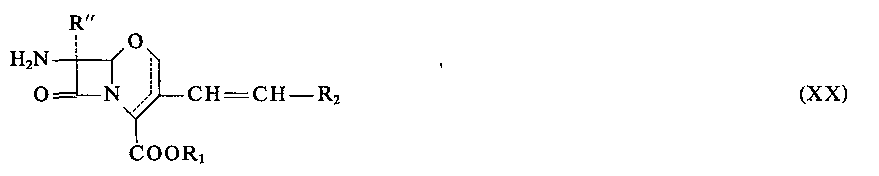 (ou sur un mélange de ses isomères),
dans laquelle R", R₁ et R₂ sont définis comme précédemment, qui se présente sous forme bicyclooctène-2 ou -3 et dont le substituant en position -3 du bicyclooctène présente la stéréoisomerie E ou Z, suivie éventuellement de l'élimination des radicaux protecteurs.

La réaction s'effectue dans les conditions décrites précédemment pour l'action d'un acide de formule générale (V) ou d'un de ses dérivés réactifs sur une amino-7 oxacéphalosporine de formule générale (VI).

Le cas échéant, l'élimination des radicaux protecteurs peut être effectuée dans les conditions décrites pour obtenir le produit de formule générale (I).

Le produit de formule générale (XX) peut être obtenu par élimination du radical protecteur R₄ d'un produit de formule générale (XVI), dans laquelle R" est situé en position 7α (ou éventuellement par élimination simultanée des radicaux protecteurs R₄ et R; lorsque l'on veut obtenir un produit de formule générale (XX) dans laquelle R₁ est hydrogène).

On opère généralement dans les conditions décrites précédemment pour l'obtention d'un produit de formule générale (VI) à partir d'un produit de formule générale (XIII).

Les produits de formule générale (XIX) peuvent être obtenus par hydrolyse de l'énamine (ou du mélange d'énamines isomères) de formule générale: 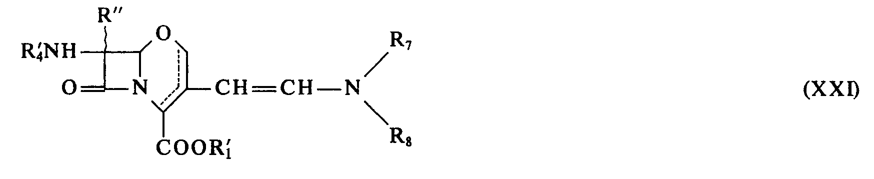 dans laquelle R", 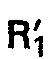 et 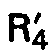 étant définis comme précédemment pour la formule générale (XIX) (étant entendu que, le cas échéant, les radicaux amino et/ou carboxy contenus par R4 sont protégés), le produit se présente sous forme bicyclooctène-2 ou -3, et le substituant sur l'atome de carbone en position -3 du bicyclooctène présente la stéréoisomérie E ou Z, et
R₇ et R₈ qui sont identiques ou différents représentent des radicaux alcoyle (éventuellement substitués par un radical alcoyloxy ou dialcoylamino) ou phényle, ou forment ensemble avec l'atome d'azote auquel ils sont rattachés un hétérocycle saturé à 5 ou 6 chaînons, contenant éventuellement un autre hétéroatome choisi parmi l'azote, l'oxygène ou le soufre et éventuellement substitué par un radical alcoyle.

On opère généralement dans un acide organique (par exemple acide formique, acide acétique) ou minéral (par exemple acide chlorhydrique, acide sulfurique) en présence ou non d'un solvant, en milieu aqueux ou organique, à une température comprise entre -20°C et la température de reflux du mélange réactionnel. Lorsque l'on opère en milieu organique, l'hydrolyse est réalisée par addition d'eau au mélange réactionnel, puis on traite éventuellement par une base minérale (par exemple bicarbonate alcalin) ou organique (par exemple amine tertiaire ou pyridine).

Lorsque l'on opère en présence d'un solvant, il n'est pas nécessaire que le solvant soit miscible à la phase aqueuse acide. Le contact est alors réalisé par agitation vive.

Parmi les solvants utilisables, peuvent être cités les solvants chlorés, l'acétate d'éthyle, letétrahydro- furannne, l'acétonitrile, le diméthylformamide, les alcools. Il n'est pas absolument nécessaire d'avoir purifié l'intermédiaire de formule générale (XXI) pour mettre en oeuvre cette réaction.

Lorsque l'on veut obtenir un aldéhyde de formule générale (XIX) dans laquelle R4 contient une fonction acide libre, il est nécessaire d'opérer à partir d'une énamine dans laquelle le groupement protecteur de la fonction acide de 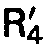 et 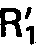 sont différents et éliminables sélectivement.

L'élimination du radical protecteurs s'effectue dans les conditions décrites précédemment.

Les produits de formule générale (XXI) peuvent être obtenus par action d'un produit de formule générale: 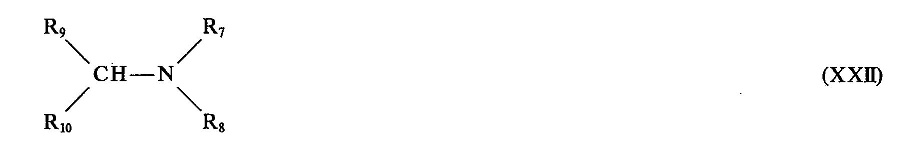 éventuellement préparé in situ, [pour lequel R₇ et R₈ sont définis comme précédemment et R₉ et R₁₀, qui sont identiques ou différents, soit représentent des groupements de formule générale: 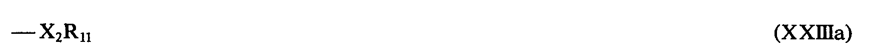 dans laquelle X₂ est un atome d'oxygène et R₁₁ représente un radical alcoyle ou phényle,
soir représentent l'un radical de formule générale (XXllla) (dans lequel X₂ représente un atome d'oxygène ou de soufre et R₁₁ est alcoyle ou phényle), et l'autre un radical amino de formule générale: 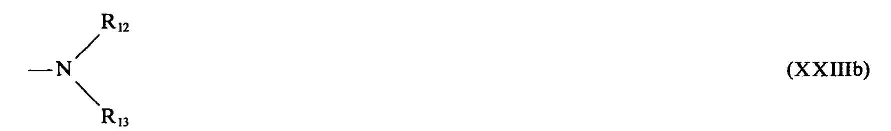 dans laquelle R₁₂et R₁₃ sont définis comme R₇ et R₈, soit encore R₉ et R₁₀ représentent chacun un radial de formule générale (XXIIIb)] sur un dérivé d'oxacéphalosporine de formule générale: 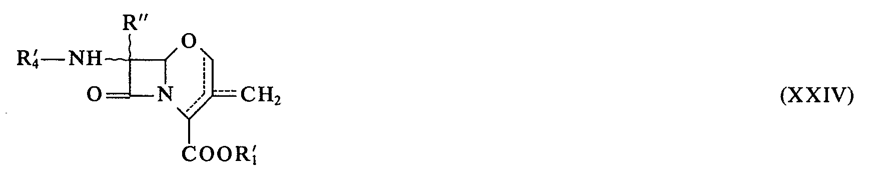 dans laquelle, R", 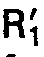 et 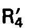 étant définis comme précédement dans la formule générale (XXI), le produit se présente sous forme méthyl-3 bicyclooctène-2 ou -3 ou méthylène-3 bicyclooctane.

Lorsque l'on choisit un produit de formule générale (XXII) dans laquelle le radical (XXlllb) est différent de-NR₇R₈, il est préférable de choisir un tel produit de manière que l'amine HNR₁₂R₁₃ soit plus volatile que HNR₇R₈.

On opère généralement dans un solvant organique tel qu'un amide (par exemple le diméthylformamide, le diméthylacétamide ou l'hexaméthylphosphorotriamide), un nitrile (par exemple l'acétonitrile), un ester (par exemple l'acétate d'éthyle), un éther (par exemple le dioxanne), un solvant chloré (par exemple le dichloro-1,2 éthane) ou encore dans un mélange de tels solvants à une température comprise entre 20°C et la température de reflux du mélange réactionnel.

II est entendu que lorsque 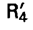 est un radical de formule générale (II) dans laquelle R^{o} est un atome d'hydrogène, il est préférable que l'oxime soit protégée dans les conditions décrites précédemment.

Il est également entendu que lorsque R4 contient un substituant hydroxy, il est préférable de protéger ce dernier.

La protection, et l'élimination des radicaux protecteurs, s'effectuent dans les conditions décrites précédemment.

Les produits de formule générale (XXII) peuvent être préparés selon les méthodes décrites par H. Bredereck et coll., Chem. Ber. 101, 41 (1968), Chem. Ber. 101, 3058 (1968) et Chem. Ber. 106, 3725 (1973).

Les dérivés de l'oxacephalosporine de formule générale (XXIV) dans laquelle R4 représente un radical de formule générale (II) ou un radical a-carboxyarylacétyle dont les fonctions amine et/ou acide sont protégées peuvent être préparés à partir des produits de formule générale: 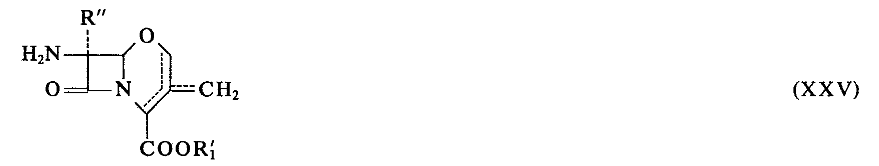 (dans laquelle 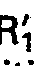 est défini comme précédemment et R" est un atome d'hydrogène ou un radical méthoxy en position a) en opérant par action d'un acide de formule générale (V) dont les radicaux amino et/ou carboxy sont protégés, ou d'un de ses dérivés, dans les conditions décrites précédemment pour l'obtention des produits de formule générale (I).

Les conditions de déblocage des substituants sont telles que décrites précédemment pour la préparation des oxacéphalosporines de formule générale (1).

Les oxacéphalosporines de formule générale (XXIV) et (XXV) peuvent être préparées par application des méthodes décrites dans la littérature; par exemple
- lorsque R" représente un atome d'hydrogène: selon les méthodes dédrites par C. L. Branch et coll., J. C. S. Perkin I, 2268 (1979); dans la demande de brevet allemand 2 806 457; dans le brevet américain 4108 992; dans la demande de brevet allemand 2 355 209 puis éventuellement mise en place du radical R4 [lorsque l'on veut obtenir un produit de formule générale (XXIV)], par analogie avec les méthodes employées en chimie des céphalosporines et par exemple:
- lorsque R4 est un radical formyle: selon J. C. Sheehan et coll., J. Amer. Chem. Soc. 80,1154 (1958),
- lorsque R4 est acétyle, chloracétyle, trichloracétyle, phénylacétyle, phénoxyacétyle ou benzoyle: selon E. H. Flynn, Cephalosporins and Penicillins, Ac. Press (1972),
- lorsque R4 est un radical t.butoxycarbonyle: selon L. Moroder et coll., Hoppe Seyler's Z. Physiol. Chem. 357, 1651 (1976),
- lorsque R4 est trichloro-2,2,2 diméthyl-1,1 éthoxycarbonyle: selon J. Ugi et coll., Angew. Chem. Int. Ed. Engl. 17(5), 361 (1978),
- lorsque R4 est trichloro-2,2,2 éthoxycarbonyle, chloro-2 diméthyl-1,1 éthoxycarbonyle, cyano-2 diméthyl-1,1 éthoxycarbonyle, triméthylsilyl-2 éthoxycarbonyle, benzyloxycarbonyle, p.méthoxy- benzyloxycarbonyle, diméthoxy-3,5 benzyloxycarbonyle, p.nitrobenzyloxycarbonyle, vinyloxycarbonyle: par action d'un chloroformiate en milieu hydroorganique en présence d'un bicarbonate alcalin, ou selon le brevet belge 788 885,
- lorsque R4 est diphénylméthoxycarbonyle: par action de l'azidoformiate correspondant en milieu hydroorganique, en présence d'un bicarbonate alcalin,
- lorsque R4 est (biphénylyl-4)-2 isopropyloxycarbonyle: par analogie avec la méthode décrite dans Helv. Chim. Acta, 51, 924 (1968),
- lorsque R4 est quinolyl-8 oxycarbonyle ou allyloxycarbonyle: par action du carbonate correspondant en milieu hydroorganique basique,
- lorsque R4 est o.nitrophényithio ou p.nitrophénylthio: par analogie avec la méthode décrite par T. Kobayashi et coll., Chem. Pharm. Bull. 27(11), 2718 (1979),
- lorsque R4NH est remplacé par diméthylaminométhylèneamino: par analogie avec la méthode décrite par J. F. Fitt, J. Org. Chem. 42(15), 2639 (1977),
- lorsque R4NH est remplacé par nitro-4 benzylidèneamino ou diméthoxy-3,4 benzylidèneamino: selon la méthode décrite par R. A. Firestone, Tetrahedron Lett., 375 (1972),
- lorsque R4NH est remplacé par di t.butyl-3,5 hydroxy-4 benzylidèneamino: selon la méthode décrite par H. Yanagisawa et coll., Tetrahedron Lett., 2705 (1975),
- lorsque R4 est bis(nitro-4 benzyl)phosphoryle: selon la méthode décrite dans la demande de brevet japonais 77 125 185,
- lorsque R" représente un radical méthoxy: par analogie avec les méthodes décrites précédemment pour la préparation des produits de formule générale (VI) ou (XIII) ou selon la demande de brevet allemand 2 806 457, puis lorsque l'on veut obtenir un produit de formule générale (XXIV) mise en place du radical R4 par analogie avec les méthodes citées ci-dessus.

Les isomères des produits de formules générales (I), (VI), (Vla), (X), (XIII), (Xllla), (XVI), XVla), (XIX), (XX), (XXI), (XXIV) et (XXV) peuvent être séparés par chromatographie ou cristallisation.

Les produits selon la présente invention peuvent être transformés en sels métalliques ou en sels d'addition avec les bases azotées selon les méthodes connues en soi. Ces sels peuvent être obtenus par action d'une base métallique (par exemple alcaline ou alcalino-terreuse), de l'ammoniac ou d'une amine sur un produit selon l'invention dans un solvant approprié tel qu'un alcool, un éther ou l'eau ou par réaction d'échange avec un sel d'un acide organique. Le sel formé précipite après concentration éventuelle de sa solution, il est séparé par filtration ou décantation. Il peut également être isolé de sa solution par lyophilisation.

Les nouveaux produits selon l'invention peuvent aussi être transformés en sels d'addition avec les acides. Selon les procédés de la présente invention les produits peuvent être obtenus sous forme de trifluoroacétate, solvate avec l'acide formique ou l'eau, phosphate, méthanesulfonate ou paratoluènesulfonate. Les produits de formule générale (I), dans laquelle R est défini selon la présente invention, obtenus sous forme de ces sels, peuvent être libérés et transformés en sels d'autres acides selon les méthodes habituelles.

Comme exemples de sels pharmaceutiquement acceptables peuvent être cités les sels avec les métaux alcalins (sodium, potassium, lithium) ou avec les métaux alcalinoterreux (magnésium, calcium), le sel d'ammonium, les sels de bases azotées (éthanolamine, diéthanolamine, triméthylamine, triéthylamine, méthylamine, propylamine, diisopropylamine, NN-diméthyléthanolamine, benzylamine, di- cyclohexylamine, N-benzyl-,8-phénéthylamine, NN'-dibenzyléthylènediamine, diphénylènediamine, benzhydrylamine, quinine, choline, arginine, lysine, leucine, dibenzylamine), ou les sels d'addition avec des acides minéraux (chlorhydrates, bromhydrates, sulfates, nitrates, phosphates) ou organiques (suc- cinates, fumarates, maléates, p.toluènesulfonates).

Les nouveaux produits selon la présente invention peuvent être éventuellement purifiés par des méthodes physiques telles que la cristallisation ou la chromatographie.

Les nouveaux dérivés de l'oxacéphalosporine selon la présente invention et leurs sels pharmaceutiquement acceptables présentent des propriétés anti-bactériennes particulièrement intéressantes. Ils manifestent une activité remarquable in vitro et in vivo sur les germes Gram-positifs et Gram-négatifs.

In vitro, les produits de formule générale (I) se sont montrés actifs à une concentration comprise entre 1 et 15 µg/cm³ sur des souches de staphylocoques sensibles à la pénicilline G (Staphylococcus aureus Smith), à une concentration comprise entre 0,01 et 1 µg/cm³ sur Escherichia coli souche NIHJ.

In vivo les produits de formule générale (I) se sont montrés actifs sur les infections expérimentales de la souris à Staphylococcus aureus Smith (sensible à la pénicilline G) à une dose comprise entre 0,5 et 15 mg/kg par jour par voie sous-cutanée, et à Escherichia coli souche NIHJ à des doses comprises entre 0,01 et 10 mg/kg par jour par voie sous-cutanée.

Par ailleurs, la DL₅₀ des produits de formule générale (I) est comprise entre 1,5 g/kg et des doses supérieures à 2,5 g/kg par voie sous-cutanée chez la souris.

D'un intérêt particulier sont les produits de formule générale (I) dans laquelle
R est dioxo-5,6 formylalcoyl-4 tétrahydro-1,4,5,6 triazine-1,2,4 yle-3 ou alcoyl-2 thiadiazol-1,3,4 yle-5,
le symbole R' est un radical de formule générale (II) dans laquelle R' est un radical alcoyle et
le symbole R" est un atome d'hydrogène,
et parmi ces produits les produits de formule générale (I) pour lesquels R' est un radical de formule générale (II) dans laquelle R' est un radical méthyle et notamment
   1'[(amino-2 thiazolyl-4)-2 méthoxyimino-2 acétamido]-7 carboxy-2 [(méthyl-2 thiadiazol-1,3,4 yl-5) thio-2 vinyl]-3 oxo-8 oxa-5 aza-1 bicyclo[4.2.0] octène-2, isomère syn, forme E 1'[(amino-2 thiazolyl-4)-2 méthoxyimino-2 acétamido]-7 carboxy-2 {[dioxo-5,6 (oxo-2 éthyl)-4 tétrahydro-1,4,5,6 triazine-1,2,4 yl-3] thio-2 vinyl}-3 oxo-8 oxa-5 aza-1 bicyclo[4.2.0] octène-2, isomère syn, forme E.

Les exemples suivants, donnés à titre non limitarif, montrent comment l'invention peut être mise en pratique.

Dans ces exemples, les produits sont cités selon la nomenclature des Chemical Abstracts. Il est entendu qu'en l'absence de mention particulière les dérivés d'oxacéphalosporine que sont cités présentent la stéréoisomérie donnée par la formule générale partielle: 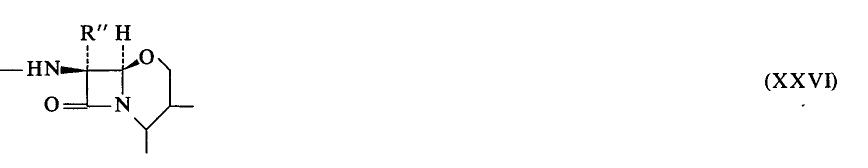 dans laquelle R" est en position α.

### Exemple 1

A une solution de 0,1 g d'amino-7 benzhydryloxycarbonyl-2 [(méthyl-2 thiadiazol-1,3,4 yl-5) thio-2 vinyl]-3 oxo-8 oxa-5 aza-1 bicyclo[4.2.0] octène-2, forme E dans 20 cm³ de dichlorométhane, on ajoute 0,089 g d'acide méthoxyimino-2 (tritylamino-2 thiazolyl-4)-2 acétique, isomère syn, 0,041 g de N,N'-dicyclohexylcarbodiimide et 0,001 g de N,N-diméthylamino-4 pyridine. Le mélange est agité à 20°C pendant 1 heure 30 minutes. On ajoute alors 0,1 cm³ d'acide acétique; on élimine un léger insoluble par filtration, concentre à sec le filtrat à 30°C sous pression réduite (100 mm de mercure; 13,3 kPa) et dissout le résidu dans 5 cm³ d'acétate d'éthyle. La solution est lavée par 2 fois 2,5 cm³ d'acide chlorhydrique 0,1 N, puis par 5 cm³ d'une solution à 1 % de bicarbonate de sodium, et par 5 cm³ d'eau distillée. On sèche sur sulfate de sodium, filtre et concentre à sec à 30°C sous 20 mm de mercure (2,7 kPa). Le résidu (0,12 g) est fixé sur 0,25 g de gel de silice Merck (0,05-0,2) et la poudre obtenue est chargée sur une colonne de 10 g de gel de silice (diamètre de la colonne: 1 cm). On élue successivement par 70 cm³ d'un mélange cyclohexane-acétate d'éthyle 50-50 (en volumes), 30 cm³ d'un mélange 40-60 et 30 cm³ d'un mélange 20-80 en recueillant des fractions de 2,5 cm³. On concentre à sec à 20°C sous pression réduite (20 mm de mercure; 2,7 kPa) les fractions 22 à 40 et recueille 0,023 g de benzhydryloxycarbonyl-2 [méthoxyimino-2 (tritylamino-2 thiazolyl-4)-2 acétamido]-7 [(méthyl-2 thiadiazol-1,3,4 yl-5) thio-2 vinyl]-3 oxo-8 oxa-5 aza-1 bicyclo[4.2.0] octène-2 isomère syn, forme E sous la forme d'une meringue orangée.

Spectre infra-rouge (KBr), bandes caractéristiques (cm⁻¹):
1795, 1720, 1685, 1520, 1495, 1450, 1210, 1045, 750, 700.

Spectre RMN du proton (350 MHz, CDCl₃, δ en ppm, J en Hz):
2,74 (s, 3 H, -CH₃); 4,07 (s, 3 H, =N-O-CH₃); 4,59 et 4,86 (2 d, J =18, 2 H, -CH₂-O-); 5,13 (d, J = 3,5, 1 H, -H en 6); 5,81 (dd, J = 3,5 et 9, 1 H, -H en 7); 6,78 (s, 1 H, -H du thiazole); 6,79 (d, J = 9, 1 H, -CO-NH-); 6,88 (s, 1 H, -COO-CH(C₆H₅)₂); 7,03 (mf, 1 H, -NH-C(C₆H₅)₃); 7,14 et 7,69 (2 d, J = 17, 2 H, -CH=CH-S-).

A une solution de 0,023 g de benzhydryloxycarbonyl-2 [méthoxyimino-2 (tritylamino-2 thiazo- lyl-4)-2 acétamido]-7 [(méthyl-2 thiadiazol-1,3,4 yl-5) thio-2 vinyl]-3 oxo-8 oxa-5 aza-1 bicyclo[4.2.0] octène-2, isomère syn, forme E, dans 1 cm³ d'acide formique, on ajoute 0,5 cm³ d'eau distillée et chauffe le mélange à 50°C sous agitation pendant 20 minutes. Après refroidissement, on filtre l'insoluble et on concentre à sec à 30°C sous pression réduite (20 mm de mercure; 2,7 kPa). On reprend le résidu par 2 fois 15 cm³ d'éthanol en concentrant à sec à chaque fois à 20°C sous 20 mm de mercure (2,7 kPa) et triture le résidu dans 20 cm³ d'éther éthylique. On obtient après filtration 0,010 g d'[(amino-2 thiazolyl-4)-2 méthoxyimino-2 acétamido]-7 carboxy-2 [(méthyl-2 thiadiazol-1,3,4 yl-5) thio-2 vinyl]-3 oxo-8 oxa-5 aza-1 bicyclo[4.2.0] octène-2, isomère syn, forme E sous la forme d'une poudre jaune.

Spectre infra-rouge (KBr), bandes caractéristiques (cm⁻¹):
1785, 1670, 1620. 1530, 1040

Spectre de RMN du proton (350 MHz, DMSO d₆, ô en ppm, J en Hz):
2,70 (s,-CH₃); 3,83 (s,=N-OCH₃); 4,50 et 4,78 (2 d, J = 18, -CH₂-O-); 5,14 (d, J = 3,5, -H en 6); 5,45 (mf,-H en 7); 6,75 (s, -H duthiazole); 7,10 à 7,70 (mt,-NH₂ et-CH=CH-S-); 9,34 (d, J = 9, -CO-NH-).

L'amino-7 benzhydryloxycarbonyl-2 [(méthyl-2 thiadiazol-1,3,4 yl-5) thio-2 vinyl]-3 oxo-8 oxa-5 aza-1 bicyclo[4.2.0] octène-2, forme E peut être obtenu de la manière suivante:
On dissout 0,112 g de benzhydryloxycarbonyl-2 [(méthyl-2 thiadiazol-1,3,4 yl-5) thio-2 vinyl]-3 oxo-8 tritylamino-7 oxa-5 aza-1 bicyclo[4.2.0] octène-2, forme E dans 1,4 cm³ d'acétone et on ajoute à la solution 0,029 g d'acide p.toluènesulfonique monohydraté. La solution est portée au reflux pendant 45 minutes pendant lesquelles des cristaux se développent sur les parois du récipient. La suspension est filtrée et le filtrat est versé dans 10 cm³ d'une solution à 1 % de bicarbonate de sodium. Le mélange est extrait par 2 fois 5 cm³ d'acétate d'éthyle, la phase organique est séchée sur sulfate de sodium, filtrée et concentrée à sec à 30°C sous pression réduite (20 mm de mercure; 2,7 kPa). On recueille ainsi 0,1 g d'un solide brut marron constitué essentiellement d'amino-7 benzhydryloxycarbonyl-2 [(méthyl-2 thiadiazol-1,3,4 yl-5) thio-2 vinyl]-3 oxo-8 oxa-5 aza-1 bicyclo[4.2.0] octène-2, forme E. Rf = 0,20 [chromatoplaque de silicagel, éluant: cyclohexane-acétate d'éthyle 80-20 (en volumes)].

La benzhydryloxycarbonyl-2 [(méthyl-2 thiadiazol-1,3,4 yl-5) thio-2 vinyl]-3 oxo-8 tritylamino-7 oxa-5 aza-1 bicyclo[4.2.0] octène-2, forme E, peut être préparé de la manière suivante:
A une solution de 2,4 g de benzhydryloxycarbonyl-2 oxo-8 (tosyloxy-2 vinyl)-3 tritylamino-7 oxa-5 aza-1 bicyclo[4.2.0] octène-2 mélange des formes E et Z, dans 15 cm³ de diméthylformamide, on ajoute 0,28 g du sel de sodium du mercapto-5 méthyl-2 thiadiazole-1,3,4. Le mélange est agité à 20°C pendant 2 heures, puis est dilué par 50 cm³ d'acétate d'éthyle. La solution est lavée par 5 fois 50 cm³ d'eau distillée et 50 cm³ d'une solution demi-saturée de chlorure de sodium, puis séchée sur sulfate de sodium. On filtre et concentre à sec à 20°C sous pression réduite (20 mm de mercure; 2,7 kPa). Le résidu (2,1 g) est chromatographié sur une colonne de gel de silice Merck (0,04-0,06) (diamètre de la colonne: 4,1 cm, hauteur: 20 cm). On élue par 1 litre d'un mélange de cyclohexane-acétate d'éthyle 70-30 (en volumes) sous une pression de 50 kPa en recueillant des fractions de 60 cm³. Les fractions 9 et 10 sont concentrées à sec sous pression réduite (30 mm de mercure; 4 kPa) à 30°C. On obtient 0,13 g de benzhydryloxycarbonyl-2[(méthyl-2 thiadiazol-1,3,4 yl-5) thio-2 vinyl]-3 oxo-8 tritylamino-7 oxa-5 aza-1 bicyclo[4.2.0] octène-2, forme E sous la forme d'une meringue jaune pâle.

Spectre infra-rouge (KBr), bandes caractéristiques (cm⁻¹):
1790,1720,1490,1450,1210,745,700

Spectre de RMN du proton (350 MHz, CDCl₃, δ en ppm, J en Hz):
2,74 (s, 3 H, -CH₃); 3,76 (d, J = 3,5, 1 H, H en 6); 4,16 et 4,62 (2 d, J = 18, 2 H, -CH₂-O-); 4,37
(d, J = 3,5, 1 H, H en 7); 6,84 (s, 1 H -COO-CH(C₆H₅)₂); 6,96 (d, J = 17, 1 H, -CH=CH-S-).

Le benzhydryloxycarbonyl-2 oxo-8 (tosyloxy-2 vinyl)-3 tritylamino-7 oxa-5 aza-1 bicyclo[4.2.0] octène-2, mélange des formes E et Z peut être préparé de la manière suivante:
On dissout 2,51 g de benzhydryloxycarbonyl-2 (oxo-2 éthyl)-3 oxo-8 tritylamino-7 oxa-5 aza-1 bicyclo[4.2.0] octène-2 dans 20 cm³ de pyridine. A la solution obtenue, on ajoute 1,13 g de chlorure de tosyle et on maintient le mélange sous agitation à 20°C pendant 1 heure 25 minutes. La solution est versée dans 150 cm³ d'eau glacée, une gomme se dépose sur les parois du récipient, la phase aqueuse est décantée et on dissout la substance gommeuse dans 45 cm³ d'acétate d'éthyle. La solution organique est lavée par 2 fois 50 cm³ d'une solution 0,1 N d'acide chlorhydrique, 50 cm³ d'une solution à 5% de bicarbonate de sodium et 30 cm³ d'une solution demi-saturée de chlorure de sodium, séchée sur sulfate de sodium, filtrée et concentrée à sec à 30°C sous pression réduite (20 mm de mercure; 2,7 kPa). On recueille 2,4 g d'un produit brut marron constitué essentiellement de benzhydryloxycarbonyl-2 oxo-8 (tosyloxy-2 vinyl)-3 tritylamino-7 oxa-5 aza-1 bicyclo[4.2.0] octène-2, mélange des formes E et Z.

Spectre infra-rouge (KBr), bandes caractéristiques (cm⁻¹):
1790, 1725, 1595, 1490, 1450, 1380, 1190, 1 180, 745, 700

Le benzhydryloxycarbonyl-2 (oxo-2 éthyl)-3 oxo-8 tritylamino-7 oxa-5 aza-1 bicyclo[4.2.0] octène-2 peut être préparé de la manière suivante:
Une solution de 3,0 g de benzhydryloxycarbonyl-2 (diméthylamino-2 vinyl)-3 oxo-8 tritylamino-7 oxa-5 aza-1 bicyclo[4.2.0] octène-2, forme E, dans 100 cm³ d'acétate d'éthyle est agitée vigoureusement pendant 1 heure 30 minutes à 20°C en présence de 45 cm³ d'une solution d'acide chlorhydrique 1 N. Le mélange est décanté et la phase organique est lavée par 50 cm³ d'une solution à 5% de bicarbonate de sodium puis par 50 cm³ d'une solution demi-saturée de chlorure de sodium. La phase acétate d'éthyle est séchée sur sulfate de sodium , filtrée et concentrée à sec à 30°C sous pression réduite (20 mm de mercure; 2,7 kPa). On obtient 2,52 g d'un produit brut constitué principalement de benzhydryloxycarbonyl-2 (oxo-2 éthyl)-3 oxo-8 tritylamino-7 oxa-5 aza-1 bicyclo[4.2.0] octène-2, sous la forme d'une meringue marron clair.

Spectre infra-rouge (KBr), bandes caractéristiques (cm⁻¹):
1790,1720,1600,1495,1450,1220,750,700

Spectre de RMN du proton (350 MHz, CDCI₃, ô en ppm, J en Hz):

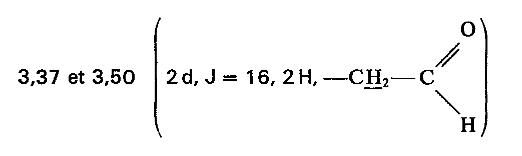

3,81 (d, J = 3,5,1 H, H en 6); 3,92 et 4,12 (2d,J = 18,2 H,-CH₂-O-);4,35 (dd,J=3,5 et 9, 1 H, H en 7); 6,80 (s, 1 H, -COO-CH(C₆H₅)₂); 9,49

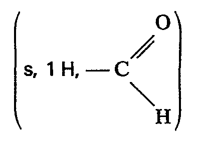

Le benzhydryloxycarbonyl-2 (diméthylamino-2 vinyl)-3 oxo-8 tritylamino-7 oxa-5 aza-1 bicyclo[4.2.0] octène-2, forme E peut être obtenu de la manière suivante:
On chauffe à 80°C sous azote, une solution de 4,25 g de benzhydryloxycarbonyl-2 méthyl-3 oxo-8 tritylamino-7 oxa-5 aza-1 bicyclo[4.2.0] octène-2 dans 20 cm³ de diméthylformamide. On ajoute, goutte à goutte en 7 minutes, dans la solution maintenue sous agitation à 80°C, 1,55 cm³ de t.butoxy bis-diméthylaminométhane. L'addition terminée, le mélange est conservé sous agitation à 80°C pendant 17 minutes. La solution est diluée par 150 cm³ d'acétate d'éthyle, la phase organique est lavée par 3 fois 60 cm³ d'eau distillée et 60 cm³ d'une solution demi-saturée de chlorure de sodium, séchée sur sulfate de sodium, filtrée et concentrée à sec à 30°C sous pression réduite (20 mm de mercure; 2,7 kPa). Le résidu est trituré dans 150 cm³ d'éther éthylique, la suspension obtenue est filtrée et le filtrat est concentré à sec à 30°C sous pression réduite (20 mm de mercure; 2,7 kPa). On obtient 3,14 g d'un produit brut constitué principalement de benzhydryloxycarbonyl-2 (diméthylamino-2 vinyl)-3 oxo-8 tritylamino-7 oxa-5 aza-1 bicyclo[4.2.0] octène-2, forme E utilisable sans purification supplémentaire.

Spectre infra-rouge (KBr), bandes caractéristiques (cm⁻¹):
1780. 1660, 1615, 1490, 1450, 745, 700.

Spectre RMN du proton (350 MHz, CDCl₃, δ en ppm, J en Hz):
2,77 (s, 6 H, -N(CH₃)_{z}); 3,71 (d, J = 3,5, 1 H, H en 6); 4,12 et 4,53 (2 d, J = 17,2 H, -CH₂-O-);
4,26 (mf, 1 H, H en 7); 6,24 et 6,40 (2 d, J = 13,2 H, -CH=CH-); 6,81 (s, 1 H, -COO-CH(C₆H₅)₂).

7,74 g de benzhydryloxycarbonyl-2 méthyl-3 oxo-8 tritylamino-7 oxa-5 aza-1 bicyclo[4.2.0] octène-2 sont préparés selon un schéma de synthèse décrit dans le brevet américain 4 108 992 dans lequel on remplace le glyoxylate de t.butyle par le glyoxylate de benzhydryle préparé selon le brevet français 1 495 047.

L'oxacéphalosporine attendue est obtenue sous la forme d'un solide blanc à partir de 13,2 g de tritylamino-3 (propyne-2 yloxy)-4 oxo-2 azétidine.

Spectre infra-rouge (KBr), bandes caractéristiques (cm⁻¹):
3340, 1780, 1715, 1620, 1595, 1585, 1490, 1450, 1220, 745, 700.

Spectre RMN du proton (350 MHz, CDCl₃, δ en ppm, J en Hz):
1,90 (s, 3 H, -CH₃); 3,75 (d, J = 3,5, 1 H, H en 6); 3,87 et 4,08 (2 d, J = 18,2 H, -CH₂-O-); 4,30 (d, J = 3,5, 1 H, H en 7); 6,85 (s, 1 H, -COO-CH(C₆H₅)₂); 7,15 à 7,4 (Mt, 26 H, aromatiques et -HN-C(C₆5_{H})₃)·

### Exemple 2

Une solution de 0,53 g de benzhydryloxycarbonyl-2 [méthoxyimino-2 (tritylamino-2 thiazolyl-4)-2 acétamido]-7 oxo-8 (tosyloxy-2 vinyl)-3 oxa-5 aza-1 bicyclo [4.2.0] octène-2, isomère syn, forme E, et de 0,139 g de [(diméthoxy-2,2 éthyl)-4 dioxo-5,6 tétrahydro-1,4,5,6 triazine-1,2,4 yl-3] thiolate de sodium dans 12 cm³ de diméthylformamide est portée à 40°C sous agitation pendant 6 heures. Le mélange est transféré dans une ampoule à décanter contenant 30 cm³ d'acétate d'éthyle et 50 cm³ d'eau distillée. Après décantation la phase organique est lavée par 3 fois 50 cm³ d'eau distillée puis séchée sur sulfate de magnésium anhydre. Le solvant est évaporé sous pression réduite (20 mm de mercure; 2,7 kPa) et le résidu (0,52 g) est purifié par chromatographie sur une colonne de gel de silice Merck (0,04-0,06) (diamètre de la colonne: 2,2 cm, hauteur: 25,5 cm). On élue par un mélange de cyclohexane-acétate d'éthyle 15-85 (en volumes) sous une pression de 50 kPa en recueillant des fractions de 20 cm³. Les fractions 10 à 28 sont concentrées à sec sous pression réduite (30 mm de mercure; 4 kPa) à 30°C. On obtient 0,20 g de benzhydryloxycarbonyl-2 ([(diméthoxy-2,2 éthyl)-4 dioxo-5,6 tétrahydro-1,4,5,6 triazine-1,2,4 yl-3] thio-2 vinyl}-3 [méthoxyimino-2 (tritylamino-2 thia- zolyl-4)-2 acétamido]-7 oxo-8 oxa-5 aza-1 bicyclo [4.2.0] octène-2, isomère syn, forme E, sous la forme d'une meringue marron clair.

Spectre de RMN du proton (250 MHz, CDCl₃, δ en ppm, J en Hz): 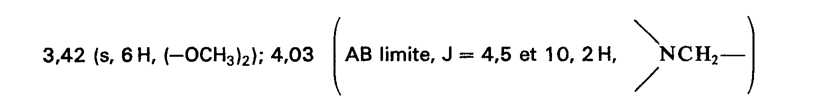
4,06 (s 3H,=N-OCH₃);4,58 (D,J=16,25,1 H,-OCH₂-);4,65 (T,J= 5,1 H,-CH(OCH₃)₂);4,84 (D,J = 16,25, 1 H,-O-CH₂-);5.15 (D, J = 3,15, 1 H,-H en 6); 5,81 (DD, J= 10 et 3,15,1 H,-H en 7); 6,73 (D, J = 16,25, 1 H, -CH=CH-); 6,79 (s, 1 H, -H du thiazole); 6,87 (s, 1 H, -COO CH(C₆H₅)₂); 7,05 (Mf, 1 H, (C₆H₅)₃ CNH-); 7,18 (D, J = 10, 1 H, -CONH-); 7,20 à 7,55 (Mf, 25 H aromatiques); 7,68 (D, J = 16,25, 1 H, -CH=CH-); 10,09 (s large, 1 H, -NH- triazine).

On ajoute 0,74 g d'acide p.toluènesulfonique monohydraté à une solution de 0,20 g de benzhydryloxycarbonyl-2 {[(diméthoxy-2,2 éthyl)-4 dioxo-5,6 tétrahydro-1,4,5,6 triazine-1,2,4 yl-3] thio-2 vinyl}-3 [méthoxyimino-2 (tritylamino-2 thiazolyl-4)-2 acétamido]-7 oxo-8 oxa-5 aza-1 bicyclo [4.2.0] octène 2, isomère syn, forme E, dans 5 cm³ d'acétonitrile à 50°C. Le mélange est maintenu à cette température pendant 30 minutes. Après refroidissement à 20°C, on filtre le précipité qui s'est développé au cours de la réaction.

Il est lavé par 1 cm³ d'acétonitrile, puis il est agité vigoureusement dans 5 cm³ d'eau distillée pendant 30 minutes. La suspension est filtrée, et la poudre marron obtenue est séchée sous pression réduite (10 mm de mercure; 1,33 kPa) donnant 20 mg de tosylate d'[amino-2 thiazolyl-4)-2 méthoxyimino-2 acétamido]-7 carboxy-2 {[dioxo-5,6 (oxo-2 éthyl)-4 tétrahydro-1,4,5,6 triazine-1,2,4 yl-3] thio-2 vinyl}-3 oxo-8 oxa-5 aza-1 bicyclo [4.2.0] octène-2, isomère syn, forme E.

Spectre de RMN du proton (250 MHz, CF₃C0₂D, ô en ppm, J en Hz):
2,47 (s, 3 H, -CH₃); 4,30 (s, 3 H, =NOCH₃); 5,17 Mf, 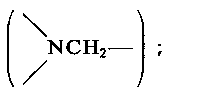 5,45 (Mf, 1 H, -H en 6); 5,91 (Mf, 1 H, -H en 7); 7,39 (D, J = 8, 2 H, H tosyle); 7,53 (s, 1 H, -H du thiazole); 7,82 (D, J = 8, 2 H, H tosyl); 9,76 (s, 1 H, -CHO).

Le benzhydryloxycarbonyl-2 [méthoxyimino-2 (tritylamino-2 thiazolyl-4)-2 acétamido]-7 oxo-8 (tosyloxy-2 vinyl)-3 oxa-5 aza-1 bicyclo[4.2.0] octène-2, isomère syn, forme E peut être préparé de la manière suivante:
On ajoute en 10 minutes 0,27 cm³ de triéthylamine à une solution refroidie à -5°C de 1,00 g de benzhydryloxycarbonyl-2 [méthoxyimino-2 (tritylamino-2 thiazolyl-4)-2 acétamido]-7 oxo-8 (oxo-2 éthyl)-3 oxa-5 aza-1 bicyclo [4.2.0] octène-2, isomère syn, et de 0,35 g de chlorure de tosyle dans 20 cm³ de dichlorométhane. On laisse sous agitation à -5°C pendant 10 minutes puis on laisse remonter à 20°C. Le mélange est alors agité pendant 30 minutes. Le solvant est évaporé sous pression réduite (350 mm de mercure, 47 kPa). On obtient ainsi une huile marron qui se solidifie par agitation avec 100 cm³ d'éther. Le solide est séparé sur filtre, et séché. On obtient 0,38 g de benzhydryloxycarbonyl-2 [méthoxyimino-2 (tritylamino-2 thiazolyl-4)-2 acétamido]-7 oxo-8 (tosyloxy-2 vinyl)-3 oxa-5 aza-1 bicyclo [4.2.0] octène-2, isomère syn, forme E, sous la forme d'une poudre de couleur crème. Le filtrat est concentré jusqu'à un volume résiduel de 5 cm³, et on ajoute 100 cm³ d'éther iso- propylique. Un solide jaune pâle se sépare. On l'isole sur filtre et obtient 0,11 g d'une deuxième fraction de benzhydryloxycarbonyl-2 [méthoxyimino-2 (tritylamino-2 thiazolyl-4)-2 acétamido]-7 oxo-8 (tosyloxy-2 vinyl)-3 oxa-5 aza-1 bicyclo [4.2.0] octène-2, isomère syn, forme E.

Spectre infra-rouge (KBr), bandes caractéristiques (cm⁻¹):
3400, 1800, 1720, 1685, 1600, 1520, 1495, 1450, 1380, 1 195, 1 180, 1070, 815, 755, 700

Spectre de RMN du proton (250 MHz, CDCl₃, δ en ppm, J en Hz):
2,44 (s, 3 H, CH₃ tosyl); 4,08 (s, 3 H, = NOCH₃); 4,44 (D, J = 16,25, 1 H, -0-CH₂-); 4,64 (D, J = 16,25, 1 H,-O-CH₂-); 5,12 (D, J = 3,75, 1 H,-H en 6); 5,79 (DD, J= 8 et 3,75,-1 H,-H en 7); 6,72 (D, J = 8,1 H,-CONH-); 6,77 (s, H,-H du thiazole); 6,82 (D, J = 12,5, H,-CH=CH-); 6,83 (s, 1 H, -COOCH(C₆H₅)₂); 7,03 (Mf, 1 H, (C₆H₅)₃ CNH-); 7,17 (D, J = 12,5, 1 H, -CH=CH-); 7,20 à 7,55 (Mf, 27 H aromatiques); 7,75 (D, J = 8, 2 H ortho du tosyle).

Le benzhydryloxycarbonyl-2 [méthoxyimino-2 (tritylamino-2 thiazolyl-4)-2 acétamido]-7 oxo-8 (oxo-2 éthyl)-3 oxa-5 aza-1 bicyclo [4.2.0] octène-2, isomère syn peut être obtenu de la manière suivante:
Une solution de 1,05 g de benzhydryloxycarbonyl-2 (diméthylamino-2 vinyl)-3 [méthoxyimino-2 (tritylamino-2 thiazolyl-4)-2 acétamido]-7 oxo-8 oxa-5 aza-1 bicyclo [4.2.0] octène-2, isomère syn, forme E dans 10 cm³ d'acétate d'éthyle est agitée vigoureusement pendant 1 heure à 20°C en présence de 5 cm³ d'une solution 1 N d'acide chlorhydrique. Le mélange est décanté et la phase organique est lavée par 10 cm³ d'une solution saturée de bicarbonate de sodium, 10 cm³ d'eau distillée et 10 cm³ d'une solution saturée de chlorure de sodium. La phase acétate d'éthyle est séchée sur sulfate de magnésium anhydre, filtrée et concentrée à sec à 30°C sous pression réduite (20 mm de mercure; 2,7 kPa). On obtient 1,00 g de benzhydryloxycarbonyl-2 [méthoxyimino-2 (tritylamino-2 thiazo- lyl-4)-2 acétamido]-7 oxo-8 (oxo-2 éthyl)-3 oxa-5 aza-1 bicyclo [4.2.0] octène-2, isomère syn sous la forme d'une meringue jaune.

Spectre infra-rouge (KBr), bandes caractéristiques (cm⁻¹):
3400, 1795, 1725, 1685, 1525, 1495, 1450, 1035, 755, 700

Spectre de masse: pic moléculaire = 8₁₇

Spectre de RMN du proton (250 MHz, CDCl₃, δ en ppm, J en Hz):
3,52 (D, J = 16,25, 1 H,-CH₂CHO); 3,62 (D, J = 16,25, H, -CH₂-CHO); 4,07 (s, 3 H,=NOCH₃); 4,35 (s, 2 H, -CH₂O-); 5,17 (D, J = 3.75, 1 H, -H en 6); 5,82 (DD, J = 10 et 3,75, 1 H, -H en 7); 6,76 (D, J = 10, 1 H,-CONH-); 6,80 (s, 1 H, -H du thiazole); 6,85 (s, 1 H, -COOCH(C₆H₅)₂; 7,13 (Mf, 1 H, (C₆H₅)₃ CNH-); 7,20 à 7,55 (Mf, 25 H, aromatiques); 9,60 (s, 1 H, -CHO).

Le benzhydryloxycarbonyl-2 (diméthylamino-2 vinyl)-3 [méthoxyimino-2 (tritylamino-2 thiazo- lyl-4)-2 acétamido]-7 oxo-8 oxa-5 aza-1 bicyclo[4.2.0] octène-2, isomère syn, forme E peut être obtenu de la manière suivante:
On chauffe à 80°C sous azote, une solution de 0,79 g de benzhydryloxycarbonyl-2 [méthoxyimino-2 (tritylamino-2 thiazolyl-4)-2 acétamido]-7 méthyl-3 oxo-8 oxa-5 aza-1 bicyclo [4.2.0] octène-2, isomère syn, dans 5 cm³ de diméthylformamide. On ajoute goutte à goutte en 6 minutes, dans la solution maintenue sous agitation à 80°C, 0,2 cm³ de t.butoxy bis-diméthylaminométhane. L'addition terminée, le mélange est conservé sous agitation à 80°C pendant 20 minutes. La solution est diluée par 25 cm³ d'acétate d'éthyle, la phase organique est lavée par trois fois 25 cm³ d'eau distillée et 25 cm³ d'une solution saturée de chlorure de sodium, séchée sur sulfate de magnésium, filtrée et concentrée à sec à 40°C sous pression réduite (20 mm dee mercure; 2,7 kPa). On obtient 0,71 g d'un produit brut constitué principalement de benzhydryloxycarbonyl-2 (diméthylamino-2 vinyl)-3 [méthoxyimino-2 (tritylamino-2 thiazolyl-4)-2 acétamido]-7 oxo-8 oxa-5 aza-1 bicyclo [4.2.0] octène-2, isomère syn, forme E.

Un échantillon (0,4 g) est purifié par chromatographie sur une colonne de silice (0,04-0,06) (diamètre de la colonne: 2,2 cm; hauteur: 20 cm) en éluant sous 50 kPa avec un mélange de cyclohexane et d'acétate d'éthyle (40-60 en volumes) et en recueillant des fractions de 40 cm³. Les fractions 9 à 15 sont rassemblées et concentrées à sec sous pression réduite (30 mm de mercure; 4 kPa) à 40°C. On obtient 0,020 g d'une poudre jaune du produit pur.

Spectre de masse: pic moléculaire = 844
Spectre de RMN du proton (350 MHz, CDCl₃, δ en ppm, J en Hz):
2,86 (s, 6 H, -N(CH₃)₂); 4,07 (s, 3 H, =NOCH₃); 4,61 et 4,76 (2 D, J = 18,2 H, -CH₂0-); 5,10 (D, J = 3,5, 1 H, H en 6); 5,69 (DD, J = 3,5 et 9, 1 H, H en 7); 6,36 et 6,54 (2 D, J=14, 2 H.-CH=CH-); 6,67 (D, J = 9, 1 H,-CONH-); 6,82 (D, 1 H, H thiazole); 6,86 (s, 1 H.-CO₂CH(C₆H₅)₂); 7,01 (s, 1 H, -NH C(C₆H₅)₃); 7,15 à 7,65 (m, 25 H)

Spectre infra-rouge (KBr), bandes caractéristiques (cm⁻¹):
1780, 1685, 1615, 1525, 1490, 1445, 1 120, 1030, 740, 695

Le benzhydryloxycarbonyl-2 [méthoxyimino-2 (tritylamino-2 thiazolyl-4)-2 acétamido]-7 méthyl-3 oxo-8 oxa-5 aza-1 bicyclo [4.2.0] octène-2, isomère syn, peut être préparé de la manière suivante: Une solution de 35 g de tosylate d'amino-7 benzhydryloxycarbonyl-2 méthyl-3 oxo-8 oxa-5 aza-1 bicyclo [4.2.0] octène-2 dans 50 cm³ d'acétate d'éthyle est lavée par 15 cm d'une solution 1 N de bicarbonate de sodium et 50 cm³ d'eau distillée puis séchée sur sulfate de magnésium. Aprèsfiltration, le solvant est évaporé sous pression réduite (20 mm de mercure; 2,7 kPa) à 40°C. On obtient 2,33 g d'amino-7 benzhydryloxycarbonyl-2 méthyl-3 oxo-8 oxa-5 aza-1 bicyclo [4.2.0] octène-2 sous forme d'une meringue jaune pâle.

Spectre de masse: pic moléculaire = 364

Spectre infra-rouge (KBr), bandes caractéristiques (cm⁻¹):
3400, 3340, 1780, 1720, 1640, 1495, 1445, 1230, 11 10, 1060, 745, 700

Spectre de RMN du proton (350 MHz, CDCl₃, δ en ppm, J en Hz):
2,03 (s, 3 H,-CH₃);4,32 (s, 2 H,-CH₂O-); 4,47 (D, J= 3,5,1 H,-H en 7); 4,92 (D, J= 3,5, 1 H,-H en 6); 6,90 (s, 1 H, -CO₂CH (C₆H₅)₂); 7,15 à 7,45 (m, 10 H aromatiques)

A une solution de 2,3 g d'amino-7 benzhydryloxycarbonyl-2 méthyl-3 oxo-8 oxa-5 aza-1 bicyclo [4.2.0] octène-2, de 2,79 g d'acide méthoxyimino-2 (tritylamino-2 thiazolyl-4)-2 acétique, isomère syn, dans 50 cm³ de chlorure de méthylène, on ajoute 1,56 g de N,N'-dicyclôhexylcarbodiimide et 0,001 g de N,N-diméthylamino-4 pyridine en solution dans 40 cm³ de chlorure de méthylène pendant 15 minutes. Le mélange est maintenu sous agitation à 0°C pendant 2 heures. Il est alors transféré dans une ampoule à décanter, puis lavé successivement par 50 cm³ d'une solution 0,1 N d'acide chlorhydrique, 50 cm³ d'eau distillée, 50 cm³ d'une solution demi-saturée de bicarbonate de sodium puis 50 cm³ d'eau distillée. La phase chlorométhylénique est séchée sur sulfate de magnésium anhydre. Le séchant filtré, la solution est concentrée à sec sous pression réduite (100 mm de mercure; 13,5 kPa). Le résidu (5,6 g) est purifié par chromatographie sur une colonne de silice (0,04-0,06) (diamètre de la colonne: 5,6 cm; hauteur: 26 cm) en éluant sous 50 kPa avec un mélange de cyclohexane et d'acétate d'éthyle (50-50 en volumes) et en recueillant des fractions de 120 cm³. Les fractions 8 à 20 sont rassemblées et concentrées à sec sous pression réduite (30 mm de mercure; 4 kPa) à 40°C. On obtient 4,02 g d'une meringue beige du benzhydryloxycarbonyl-2 [méthoxyimino-2 (tritylamino-2 thiazolyl-4)-2 acétamido]-7 méthyl-3 oxo-8 oxa-5 aza-1 bicyclo [4.2.0] octène-2, isomère syn, pur.

Spectre de masse: pic moléculaire = 789

Spectre infra-rouge (KBr), bandes caractéristiques (cm⁻¹):
2820, 1790, 1745, 1580, 1530, 1495, 1450, 1 165, 1040, 750, 700

Spectre de RMN du proton (350 MHz, CDCl₃, δ en ppm, J en Hz):
2,04 (s, 3 H,-CH₃); 4,07 (s, 3 H,=N-OCH₃); 4,32 (s, 2 H,-CH₂O-); 5,12 (D, J = 3,5,1 H,-H en 6); 5,75 (DD, J = 3,5 et 9,1 H, -H en 7); 6,68 (D, J = 9,1 H, -CONH-); 6,79 (s, 1 H, H thiazole); 6,89 (s, 1 H, -CO₂CH (C₆H₅)₂); 7,00 (s large, 1 H, -NH C(C₆H₅)₃); 7,15 à 7,55 (m, 25 H aromatiques).

Le tosylate d'amino-7 benzhydryloxycarbonyl-2 méthyl-3 oxo-8 oxa-5 aza-1 bicyclo [4.2.0] octène-2 peut être obtenu de la manière suivante:
On agite à 40°C pendant 30 minutes une solution de 6,07 g de benzhydryloxycarbonyl-2 méthyl-3 oxo-8 tritylamino-7 oxa-5 aza-1 bicyclo [4.2.0] octène-2 et 1,9 g d'acide p.toluènesulfonique hydraté dans 100 cm³ d'acétone. Le mélange est refroidi à 0°C et un solide blanc précipite. On essore et on lave le gâteau par deux fois 5 cm³ d'acétone. On obtient ainsi 3,17 g de tosylate d'amino-7 benzhydryloxycarbonyl-2 méthyl-3 oxo-8 oxa-5 aza-1 bicyclo [4.2.0] octène-2.

Le filtrat acétonique est concentré à sec sous pression réduite (20 mm de mercure; 2,7 kPa). Le résidu est trituré dans deux fois 100 cm³ d'éther éthylique. L'éther est décanté et le solide obtenu est repris dans 20 cm³ d'acétate d'éthyle. On filtre et on obtient une deuxième fraction de 0,72 g de tosylate d'amino-7 benzhydryloxycarbonyl-2 méthyl-3 oxo-8 oxa-5 aza-1 bicyclo [4.2.0] octène-2.

Spectre infra-rouge (CHBr₃), bandes caractéristiques (cm-¹):
3300, 2400, 1800, 1720, 1500, 1455, 1225, 820, 760, 745, 570

Spectre de RMN du proton (350 MHz, CDCI₃, α en ppm, J en Hz):
1,73 (s, 3 H, -CH₃); 2,15 (s, 3 H, -CH₃ (APTS)); 3,98 (D, J = 19, 1 H, -CH₂0-); 4,11 (D, J = 19, 1 H, -CH₂0-); 4,78 (D, J = 2,5, 1 H, -H en 7); 4,86 (D, J = 2,5, 1 H, -H en 6); 6,87 (s, 1 H, -COOCH (C₆H₅)₂); 6,96 (D, J = 7,5, 1 H, -H en ortho du méthyle, APTS); 7,10 à 7,60 (Mt, 10 H aromatiques); 7,74 (D, J = 7,5, 1 H, -H en ortho du S0₃H, APTS); 8,60 (Mf, 3 H, -NH3').

La présente invention concerne également les médicaments qui contiennent comme produit actif au moins un produit de formule générale (I) à l'état pur (sous forme libre ou sous forme de sel) ou sous forme d'une composition en association avec un ou plusieurs adjuvants pharmaceutiquement acceptables. Ces médicaments peuvent être utilisés par voie orale, parentérale ou rectale.

Comme compositions solides pour administration orale peuvent être utilisés des comprimés, des pilules, des poudres ou des granulés. Dans ces compositions, le produit actif selon l'invention est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale peuvent être des solutions stériles aqueuses ou non aqueuses, des suspensions ou des émulsions. Comme solvant ou véhicule, on peut employer le propylèneglycol, un polyéthylèneglycol, des huiles végétales, en particulier l'huile d'olive et des esters organiques injectables, par exemple l'oléate d'éthyle. Ces compositions peuvent également contenir des adjuvants, en particulier des agents mouillants, émulsifiants ou dispersants. La stérilisation peut se faire de plusieurs façons, par exemple à l'aide d'un filtre bactériologique, en incorporant à la composition des agents stérilisants, par irradiation ou par chauffage. Elles peuvent être également préparées sous forme de compositions solides stériles qui seront dissoutes au moment de l'emploi dans de l'eau stérile ou tout autre milieu stérile injectable.

Les compositions pour administration rectale sont des suppositoires qui peuvent contenir, outre le produit actif, des excipients tels que le beurre de cacao ou des glycérides semi-synthétiques.

En thérapeutique humaine, les médicaments selon la présente invention sont particulièrement utiles dans le traitement des infections d'origine bactérienne.

D'une façon générale, le médecin déterminera la posologie qu'il estime la plus appropriée en fonction de l'âge, du poids, du degré de l'infection et des autres facteurs propres au sujet à traiter. Généralement, les doses sont comprises entre 1 et 10 g par jour de produit actif par voie orale, intramusculaire ou intraveineuse pour un adulte.

L'exemple suivant, donné à titre non limitarif, illustre une composition selon la présente invention.

### Exemple

On prépare 100 cm³ d'une solution aqueuse isotonique contenant 1,61 g de bicarbonate de sodium et, comme produit actif, 10 g d'[(amino-2 thiazolyl-4)-2 méthoxyimino-2 acétamido]-7 carboxy-2 [(méthyl-2 thiadiazol-1,3,4 yl-5) thio-2 vinyl]-3 oxo-8 oxa-5 aza-1 bicyclo[4.2.0] octène-2, isomère syn, forme E. Après filtration sur filtre bactériologique, on répartit aseptiquement cette solution en ampoules (à raison de 10 cm³ par ampoule), on lyophilise et on scelle les ampoules.

Chaque ampoule contient l'équivalent de 1 g du produit actif, sous forme de son sel de sodium.

## Revendications (Revendications pour l'(les) Etat(s) contractant(s) suivant(s) : BE CH DE FR GB IT LI LU NL SE)

1. Une oxacéphalosporine de formule générale: 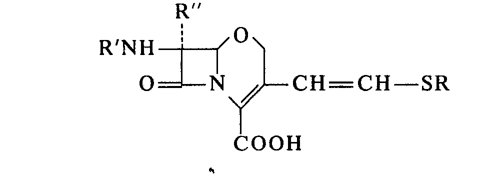 dans laquelle:
le symbole R est choisi parmi
1 ) dioxo-5,6 tétrahydro-1,4,5,6 triazine-1,2,4 yle-3 substitué en position -4 par
a) un radical alcoyle contenant 1 ou 2 atomes de carbone éventuellement substitué par un radical alcoyloxy, alcoylthio, formyle,
b) un radical allyle, dihydroxy-2,3 propyle, dihydroxy-1,3 propyle-2 ou formyl-2 hydroxy-2 éthyle,
c) un radical alcoyle contenant 2 ou 3 atomes de carbone substitué par hydroxy, carbamoyloxy, alcanoyloxy ou alcanoylamino (dont les portions alcanoyles sont non substituées ou substituées par amino), alcoylsulfonylamino, uréido, alcoyluréido ou dialcoyluréido,
2) dioxo-5,6 tétrahydro-1,4,5,6 triazine-1,2,4 yle-3 substitué en position -1 par un radical alcoyle contenant 1 ou 2 atomes de carbone éventuellement substitué par un radical formyle,
3) thiadiazol-1,3,4 yle-5 substitué par alcoyle, dialcoylaminoalcoyle ou alcanoylaminoalcoyle,
le symbole R' représente un radical de formule générale: 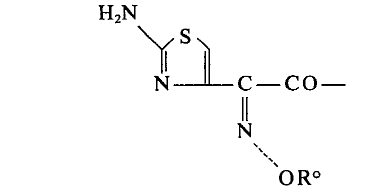 [dans laquelle R° est un atome d'hydrogène, un radical alcoyle, vinyle, ou carboxyalcoyle représenté par la formule générale: 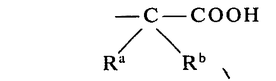 dans laquelle les radicaux R^{a} et R^{b}, qui sont identiques ou différents, représentent des atomes d'hydrogène ou des radicaux alcoyle, ou forment ensemble un radical alcoylène contenant 2 ou 3 atomes de carbone], ou R' représente un radical a-carboxyarylacétyle dans lequel aryle est un radical phényle (éventuellement substitué par un radical p.hydroxy) ou un radical thiényle-2 ou -3, et
le symbole R" représente un atome d'hydrogène ou un radical méthoxy en position 7a, étant entendu que les radicaux et portions alcoyles ou alcanoyles cités ci-dessus sont (sauf mention spéciale) droits ou ramifiés et contiennent 1 à 4 atomes de carbone,
sous ses formes E ou Z et leurs mélanges,
le cas échéant sous ses formes syn ou anti ou leurs mélanges ainsi que ses sels métalliques, ses sels d'addition avec les bases azotées et lorsqu-ils existent ses sels d'addition avec les acides.

2. Un produit selon la revendication 1 caractérisé en ce que le symbole R est un radical dioxo-5,6 formylalcoyl-4 tétrahydro-1,4.5,6 triazine-1,2,4yle-3 ou alcoyl-2 thiadiazol-1,3,4 yle-5, le symbole R' est un radical de formule générale (II) dans laquelle R° est un radical alcoyle et le symbole R" est un atome d'hydrogène, sous ses formes syn ou anti et E ou Z ou leurs mélanges, ainsi que ses sels métalliques, ses sels d'addition avec les bases azotées et ses sels d'addition avec les acides.

3. L-[(amino-2 thiazolyl-4)-2 méthoxyimino-2 acétamido]-7 carboxy-2 [(méthyl-2 thiadiazol-1,3,4 yl-5) thio-2 vinyl]-3 oxo-8 oxa-5 aza-1 bicyclo[4.2.0] octène-2 isomère syn, forme E.

4. L'[(amino-2 thiazolyl-4)-2 méthoxyimino-2 acétamido]-7 carboxy-2 {[dioxo-5,6 (oxo-2 éthyl)-4 tétrahydro-1,4,5,6 triazine-1,2,4 yl-3] thio-2 vinyl)-3 oxo-8 oxa-5 aza-1 bicyclo[4.2.0] octène-2, isomère syn, forme E.

5. Procédé de préparation d'un produit selon la revendication 1, caractérisé en ce que l'on fait agir un acide de formule générale: 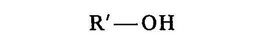 dans laquelle R' est défini comme dans la revendication 1 (étant entendu que, le cas échéant, la fonction amine et l'oxime contenues dans ce radical sont protégées), ou un dérivé réactif de cet acide, sur une amino-7 oxacéphalosporine de formule générale: 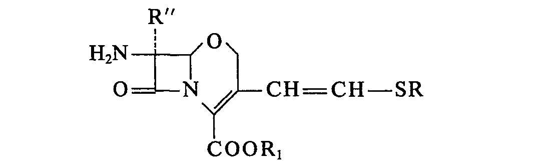 dans laquelle R" et R sont définis comme dans la revendication 1 et R, représente un atome d'hydrogène ou un radical protecteur, puis élimine les radicaux protecteurs et transforme éventuellement le produit obtenu en un sel métallique, en un sel d'addition avec une base azotée ou en un sel d'addition avec un acide.

6. Procédé selon la revendication 5, caractérisé en ce qu'on utilise un acide R'OH dans lequel R' est un radical de formule générale: 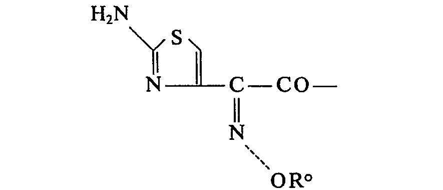 dans laquelle R° est défini selon la revendication 1, et dont la fonction amine est préalablement protégée par un groupement t.butoxycarbonyle, trichloro-2,2,2 éthoxycarbonyle, trichloracétyle, chloracétyle, trityle, dibenzyle, benzyle, benzyloxycarbonyle, p.nitrobenzyloxycarbonyle, p.méthoxybenzyloxy- carbonyle, formyle ou trifluoracétyle.

7. Procédé selon la revendication 5, caractérisé en ce qu'on utilise un acide R'OH dans lequel R' est un radical de formule générale: 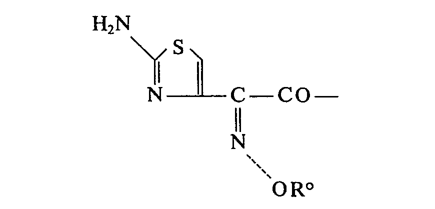 dans laquelle R° est un groupement trityle, tétrahydropyrannyle ou méthoxy-2 propyle-2 protecteur de la fonction oxime.

8. Procédé selon la revendication 5, caractérisé en ce qu'on utilise un acide R'OH dans lequel R' est un radical de formule générale: 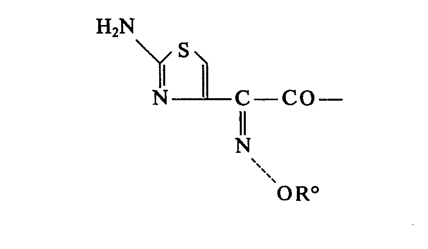 dans laquelle R° est un groupement carboxyalcoyle (représenté par la formule générale: 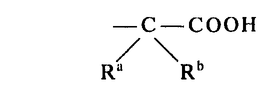 dans laquelle R^{a} et R^{b} sont définis comme dans la revendication 1 ) préalablement protégé par un radical choisi parmi méthoxyméthyle, t.butyle, benzhydryle, benzyle, nitrobenzyle et p.méthoxybenzyle.

9. Procédé selon la revendication 5, caractérisé en ce qu'on utilise une amino-7 oxacéphalosporine dans laquelle le radical facilement éliminable représenté par R₁ est choisi parmi les radicaux méthoxyméthyle, t.butyle, benzhydryle, benzyle, nitrobenzyle et p.méthoxybenzyle.

10. Procédé selon la revendication 5, caractérisé en ce qu'on utilise un dérivé réactif de l'acide de formule générale: 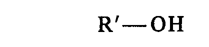 choisi parmi l'anhydride, un anhydride mixte, un ester réactif et un halogénure de cet acide.

11. Procédé de préparation d'un produit selon la revendication 1 caractérisé en ce que l'on fait agir un thiol (libre ou à l'état de sel alcalin ou alcalino-terreux) de formule générale: 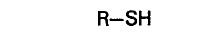 {dans laquelle le substituant de R, qui est défini comme dans la revendication 1, est protégé à l'état d'acétal [défini par les formules générales 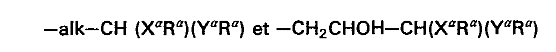 dans lesquelles alk est un radical alcoylène contenant 1 à 4 atomes de carbone, X" et Y" sont identiques et représentent des atomes d'oxygène ou de soufre et R" représente un radical alcoyle, ou bien X" et Y" sont identiques ou différents et représentent des atomes d'oxygène ou de soufre et les radicaux R" forment ensemble un radical alcoylène contenant 2 ou 3 atomes de carbone], lorsque l'on veut obtenir un produit selon la revendication 1 pour lequel R contient un radical formyle}, sur un dérivé d'oxacéphalosporine (ou, le cas échéant, sur un mélange des isomères) de formule générale: 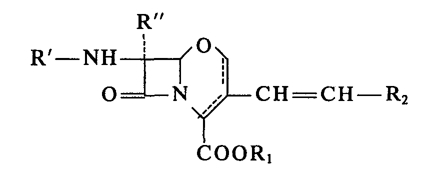 qui se présente sous forme bicyclooctène-2 ou -3 et dans laquelle, R' et R" étant définis selon la revendication 1, R, étant défini selon la revendication 5,
le substituant sur l'atome de carbone en position -3 du bicyclooctène présente la stéréoisomérie E ou Z et R₂ représente un radical de formules générales: 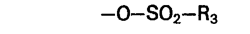 ou 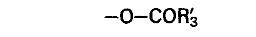 [dans lesquelles R₃ est un radical alcoyle droit ou ramifié contenant 1 à 4 atomes de carbone, trifluorométhyle, trichlorométhyle ou phényle non substitué ou substitué par un atome d'halogène ou par un radical alcoyle ou nitro, et
R3 est défini comme R₃ ou représente un radical alcanoylméthyle, alcanoyl-2 éthyle, alcanoyl-2 propyle, alcoyloxycarbonylméthyle, alcoyloxycarbonyl-2 éthyle ou alcoyloxycarbonyl-2 propyle], ou un atome d'halogène, puis on élimine s'il y a lieu les radicaux protecteurs et transforme éventuellement le produit obtenu en un sel métallique, en un sel d'addition avec une base azotée ou en un sel d'addition avec un acide.

12. Procédé selon la revendication 11, caractérisé en ce que la réaction est effectuée en présence d'une pyridine ou d'une base organique tertiaire.

13. Composition pharmaceutique caractérisée en ce qu'elle contient au moins un produit selon la revendication 1,
en association avec un ou plusieurs diluants ou adjuvants compatibles et pharmaceutiquement acceptables.

## Revendications (Revendications pour l'(les) Etat(s) contractant(s) suivant(s) : AT)

Procédé de préparation d'une oxacéphalosporine de formule générale: 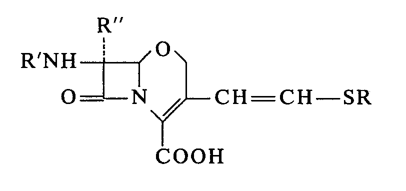 dans laquelle:
le symbole R est choisi parmi
1 ) dioxo-5,6 tétrahydro-1,4,5,6 triazine-1,2,4 yle-3 substitué en position -4 par
a) un radical alcoyle contenant 1 ou 2 atomes de carbone éventuellement substitué par un radical alcoyloxy, alcoylthio, formyle,
b) un radical allyle, dihydroxy-2,3 propyle, dihydroxy-1,3 propyle-2 ou formyl-2 hydroxy-2 éthyle,
c) un radical alcoyle contenant 2 ou 3 atomes de carbone substitué par hydroxy, carbamoyloxy, alcanoyloxy ou alcanoylamino (dont les portions alcanoyles sont non substituées ou substituées par amino), alcoylsulfonylamino, uréido, alcoyluréido ou dialcoyluréido,
2) dioxo-5,6 tétrahydro-1,4,5,6 triazine-1,2,4 yle-3 substitué en position -1 par un radical alcoyle contenant 1 ou 2 atomes de carbone éventuellement substitué par un radical formyle,
3) thiadiazol-1,3,4 yle-5 substitué par alcoyle, dialcoylaminoalcoyle ou alcanoylaminoalcoyle,
le symbole R' représente un radical de formule générale: 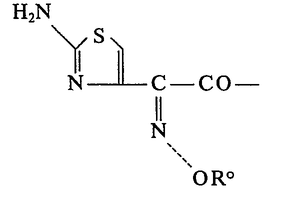 [dans laquelle R° est un atome d'hydrogène, un radical alcoyle, vinyle, ou carboxyalcoyle représenté par la formule générale: 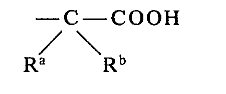 dans laquelle les radicaux R^{a} et R^{b}, qui sont identiques ou différents, représentent des atomes d'hydrogène ou des radicaux alcoyle, ou forment ensemble un radical alcoylène contenant 2 ou 3 atomes de carbone], ou R' représente un radical a-carboxyarylacétyle dans lequel aryle est un radical phényle (éventuellement substitué par un radical p.hydroxy) ou un radical thiényle-2 ou -3, et
le symbole R" représente un atome d'hydrogène ou un radical méthoxy en position 7a, étant entendu que les radicaux et portions alcoyles ou alcanoyles cités ci-dessus sont (sauf mention spéciale) droits ou ramifiés et contiennent 1 à 4 atomes de carbone,
sous ses formes E ou Z et leurs mélanges,
le cas échéant sous ses formes syn ou anti ou leurs mélanges ainsi que ses sels métalliques, ses sels d'addition avec les bases azotées et lorsqu'ils existent ses sels d'addition avec les acides, caractérisé en ce que l'on fait agir un acide de formule générale: 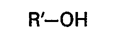 dans laquelle R' est défini comme précédemment (étant entendu que, le cas échéant, la fonction amine et l'oxime contenues dans ce radical sont protégées, ou un dérivé réactif de cet acide, sur une amino-7 oxacéphalosporine de formule générale: 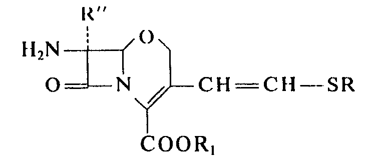 dans laquelle R" et R sont définis comme précédemment et R₁ représente un atome d'hydrogène, ou un radical protecteur, puis élimine les radicaux protecteurs et transforme éventuellement le produit obtenu en un sel métallique, en un sel d'addition avec une base azotée ou en un sel d'addition avec un acide, ou en ce que l'on fait agir un thiol (libre ou à l'état de sel alcalin ou alcalino-terreux) de formule générale: 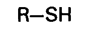 {dans laquelle le substituant de R, qui est défini comme ci-dessus, est protégé à l'état d'acétal [défini par les formules générales 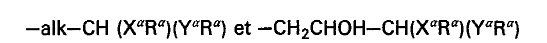 dans lesquelles alk est un radical alcoylène contenant 1 à 4 atomes de carbone, X" et Y" sont identiques et représentent des atomes d'oxygène ou de soufre et R" représente un radical alcoyle, ou bien X" et Y" sont identiques ou différents et représentent des atomes d'oxygène ou de soufre et les radicaux R" forment ensemble un radical alcoylène contenant 2 ou 3 atomes de carbone], lorsque l'on veut obtenir un produit pour lequel R contient un radical formyle}, sur un dérivé d'oxacéphalosporine (ou, le cas échéant, sur un mélange des isomères) de formule générale: 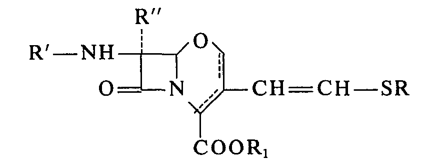 qui se présente sous forme bicyclooctène-2 ou -3 et dans laquelle, R₁, R' et R" étant définis comme ci-dessus,
le substituant sur l'atome de carbone en position -3 du bicyclooctène présente la stéréoisomérie E ou Z et R₂ représente un radical de formules générales: 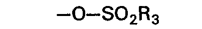 ou 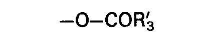 [dans lesquelles R₃ est un radical alcoyle droit ou ramifié contenant 1 à 4 atomes de carbone, trifluorométhyle, trichlorométhyle ou phényle non substitué ou substitué par un atome d'halogène ou par un radical alcoyle ou nitro, et
R3 est défini comme R₃ ou représente un radical alcanoylméthyle, alcanoyl-2 éthyle, alcanoyl-2 propyle, alcoyloxycarbonylméthyle, alcoyloxycarbonyl-2 éthyle ou alcoyloxycarbonyl-2 propyle], ou un atome d'halogène, puis on élimine s'il y a lieu les radicaux protecteurs et transforme éventuellement le produit obtenu en un sel métallique, en un sel d'addition avec une base azotée ou en un sel d'addition avec un acide.

## Patentansprüche (Patentansprüche für folgende(n) Vertragsstaat(en) : BE, CH, DE, FR, GB, IT, LI, LU, NL, SE)

1. Ein Oxacephalosporin der allgemeinen Formel 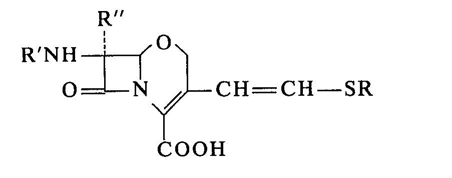 worin das Symbol R ausgewählt ist unter
1) 5,6-Dioxo-1,4,5,6-tetrahydro-1,2,4-triazin-3-yl substituiert in 4-Stellung durch
a) einen Alkylrest mit 1 oder 2 Kohlenstoffatomen, gegebenenfalls substituiert durch einen Alkyloxy-, Alkylthio-, Formylrest,
b) einen Allyl-, 2,3-Dihydroxypropyl-, 1,3-Dihydroxypropyl-2- oder 2-Formyl-2-hydroxyäthyl- rest,
c) einen Alkylrest mit 2 oder 3 Kohlenstoffatomen, substituiert durch Hydroxy, Carbamoyloxy, Alkanoyloxy oder Alkanoylamino (dessen Alkanoylteile nicht substituiert oder substituiert durch Amino sind), Alkylsulfonylamino, Ureido, Alkylureido oder Dialkylureido,
2) 5,6-Dioxo-1,4,5,6-tetrahydro-1,2,4-triazin-yl-3, substituiert in 1 -Stellung durch einen Alkylrest mit 1 oder 2 Kohlenstoffatomen, gegebenenfalls substituiert durch einen Formylrest,
3) 1,3,4-Thiadiazol-yl-5, substituiert durch Alkyl, Dialkylaminoalkyl oder Alkanoylaminoalkyl,
das Symbol R' einen Rest der allgemeinen Formel 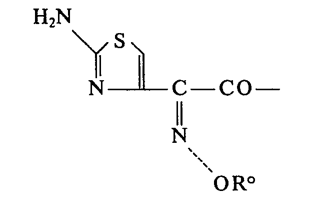 bedeutet, [worin R^{O} ein Wasserstoffatom, ein Alkyl-, Vinyl- oder Carboxyalkylrest, dargestellt durch die allgemeine Formel 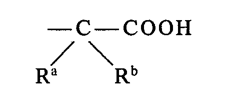 ist, worin die Reste R^{a} und R^{b}, welche identisch oder verschieden sind, Wasserstoffatome oder Alkylreste bedeuten oder zusammen einen Alkylenrest mit 2 oder 3 Kohlenstoffatomen bilden] oder
R' bedeutet einen α-Carboxyarylacetylrest, worin Aryl ein Phenylrest (gegebenenfalls substituiert durch einen p-Hydroxyrest) oder ein Thienyl-2- oder -3-rest ist und
das Symbol R" bedeutet ein Wasserstoffatom oder einen Methoxyrest in 7a-Stellung, wobei die erwähnten Alkyl- oder Alkanoylreste und -teile gerade oder verzweigt sind (wenn nichts anderes angegeben ist) und 1 bis 4 Kohlenstoffatome enthalten,
in seinen E- oder Z-Formen und deren Gemischen
gegebenenfalls in seinen syn- oder anti-Formen und deren Gemischen sowie seine Metallsalze, seine Additionssalze mit Stickstoffbasen und, falls existent, seine Additionssalze mit Säuren.

2. Ein Produkt gemäß Anspruch 1, dadurch gekennzeichnet, daß das Symbol R ein 5,6-Dioxo-4-for- mylalkyl-1,4,5,6-tetrahydro-1,2,4-triazin-yl-3- oder 2-Alkyl-1,3,4-thiadiazol-yl-5-rest ist, das Symbol R' ein Rest der allgemeinen Formel (II) ist, worin R^{o}ein Alkylrest ist und das Symbol R" ein Wasserstoffatom ist, in seinen syn- oder anti-Formen und E- oder Z-Formen und deren Gemische, sowie seine Metallsalze, seine Additionssalze mit Stickstoffbasen und seine Additionssalze mit Säuren.

3. 7-[2-(2-Aminothiazolyl-4)-2-methoxyimino-acetamido]-2-carboxy-3-[2-(2-methyl-1,3,4-thiadiazol-yl-5)-thiovinyl]-8-oxo-5-oxa-1 -aza-bicyclo[4.2.0]octen-2,syn-Isomeres, Form E.

4. 7-[2-(2-Aminothiazolyl-4)-2-methoxyimino-acetamido]-2-carboxy-3-(2-[5,6-dioxo-4-(2-oxo- äthyl)-1,4,5,6-tetrahydro-1,2,4-triazin-yl-3]-thio-vinyl}-8-oxo-5-oxa-1-aza-bicyclo[4.2.0]octen-2,syn-lsomeres, Form E.

5. Verfahren zur Herstellung eines Produkts gemäß Anspruch 1, dadurch gekennzeichnet, daß man eine Säure der allgemeinen Formel 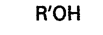 worin R' wie in Anspruch 1 definiert ist (wobei gegebenenfalls die Aminfunktion und das Oxim, welche in diesem Rest enthalten sind, geschützt werden) oder ein reaktives Derivat einer solchen Säure auf ein 7-Amino-oxacephaiosporin der allgemeinen Formel 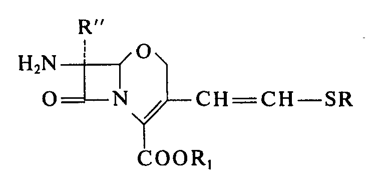 worin R" und R wie in Anspruch 1 definiert sind und R₁ ein Wasserstoffatom oder einen Schutzrest darstellt, einwirken läßt, dann die Schutzgruppen entfernt und gegebenenfalls das erhaltene Produkt in ein Metallsalz, in ein Additionssalz mit einer Stickstoffbase oder in ein Additionssalz mit einer Säure überführt.

6. Verfahren gemäß Anspruch 5, dadurch gekennzeichnet, daß man eine Säure R'OH verwendet, worin R' ein Rest der allgemeinen Formel 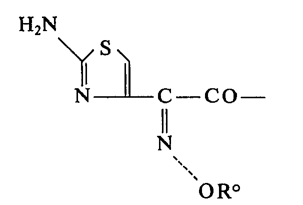 ist, worin R° gemäß Anspruch 1 definiert ist und dessen Aminfunktion vorher durch eine tert.-Butoxycarbonyl-, 2,2,2-Trichloräthoxycarbonyl-, Trichloracetyl-, Chloracetyl-, Trityl-, Dibenzyl-, Benzyl-, Benzyloxycarbonyl-, p-Nitrobenzyloxycarbonyl-, p-Methoxybenzyloxycarbonyl-, Formyl- oder Trifluoracetylgruppe geschützt wurde.

7. Verfahren gemäß Anspruch 5, dadurch gekennzeichnet, daß man eine Säure R'OH verwendet, worin R' ein Rest der allgemeinen Formel 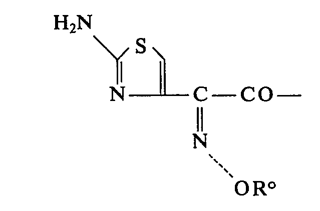 ist, worin R° eine Trityl-, Tetrahydropyranyl- oder 2-Methoxypropyl-2-Schutzgruppe der Oximfunktion ist.

8. Verfahren gemäß Anspruch 5, dadurch gekennzeichnet, daß man eine Säure R'OH verwendet, worin R' ein Rest der allgemeinen Formel 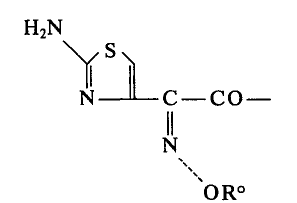 ist, worin R° eine Carboxyalkylgruppe (dargestellt durch die allgemeine Formel 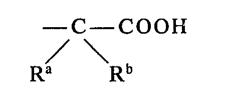 worin R^{a} und R^{b} wie in Anspruch 1 definiert sind) ist, die vorher durch einen Rest, ausgewählt unter Methoxymethyl, tert.-Butyl, Benzhydryl, Benzyl, Nitrobenzyl und p-Methoxybenzyl, geschützt ist.

9. Verfahren gemäß Anspruch 5, dadurch gekennzeichnet, daß man ein 7-Amino-oxacephalosporin verwendet, worin der leicht entfernbare, durch R₁ dargestellte Rest ausgewählt ist unter den Methoxymethyl-, tert.-Butyl, Benzhydryl-, Benzyl-, Nitrobenzyl- und p-Methoxybenzylresten.

10. Verfahren gemäß Anspruch 5, dadurch gekennzeichnet, daß man ein reaktives Derivat der Säure der allgemeinen Formel 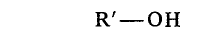 verwendet, ausgewählt unter Anhydrid, einem gemischten Anhydrid, einem reaktiven Ester und einem Halogenid dieser Säure.

11. Verfahren zur Herstellung eines Produkts gemäß Anspruch 1, dadurch gekennzeichnet, daß man ein Thiol (frei oder im Zustand des Alkali- oder Erdalkalisalzes) der allgemeinen Formel 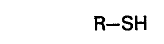 {worin der Substituent von R, der wie in Anspruch 1 definiert ist, im Acetalzustand geschützt ist [definiert durch die allgemeinen Formeln 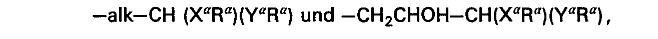 worin alk ein Alkylenrest mit 1 bis 4 Kohlenstoffatomen, X" und Y" identisch sind und Sauerstoff- oder Schwefelatome bedeuten und R^{α} einen Alkylrest bedeutet oder X^{α} und Y^{α} sind identisch oder verschieden und bedeuten Sauerstoff- oder Schwefelatome und die Reste R" bilden zusammen einen Alkylenrest mit 2 oder 3 Kohlenstoffatomen], falls man ein Produkt gemäß Anspruch 1 erhalten will, wofür R einen Formylrest enthält), auf ein Oxacephalosporinderivat (oder gegebenenfalls auf ein Gemisch der Isomeren) der allgemeinen Formel 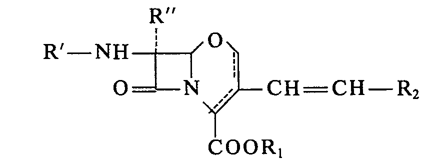 einwirken läßt, das in Bicycloocten-2- oder -3-Form vorliegt und worin R' und R" gemäß Anspruch 1 definiert sind, wobei R₁ gemäß Anspruch 5 definiert ist.
der Substituent am Kohlenstoffatom in 3-Stellung des Bicyclooctens die E- oder Z-Stereoisomerie aufweist und R₂ einen Rest der allgemeinen Formeln 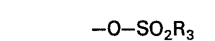 oder 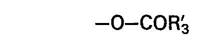 bedeutet [worin R₃ ein gerader oder verzweigter Alkylrest mit 1 bis 4 Kohlenstoffatomen, Trifluormethyl, Trichlormethyl oder Phenyl, nicht substituiert oder substituiert durch ein Halogenatom oder durch einen Alkyl- oder Nitrorest, ist und 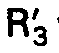 wie R₃ definiert ist oder einen Alkanoylmethyl-, 2-Alkanoyläthyl-, 2-Alkanoyl-propyl-, Alkyloxycarbonylmethyl-, 2-Alkyloxycarbonyläthyl- oder 2-Alkyloxycarbonyl-propyl-rest bedeutet] oder ein Halogenatom bedeutet, daß man dann gegebenenfalls die Schutzgruppen entfernt und gegebenenfalls das erhaltene Produkt in ein Metallsalz, in ein Additionssalz mit einer Stickstoffbase oder in ein Additionssalz mit einer Säure überführt.

12. Verfahren gemäß Anspruch 11, dadurch gekennzeichnet, daß die Reaktion in Gegenwart eines Pyridins oder einer tertiären organischen Base durchgeführt wird.

13. Pharmazeutische Zusammensetzung, dadurch gekennzeichnet, daß sie mindestens ein Produkt gemäß Anspruch 1, zusammen mit einem oder mehreren verträglichen und pharmazeutisch annehmbaren Verdünnungs- oder Hilfsmitteln enthält.

## Patentansprüche (Patentansprüche für folgende(n) Vertragsstaat(en) : AT)

Verfahren zur Herstellung eines Oxacephalosporins der allgemeinen Formel 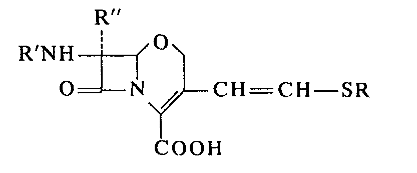 worin
das Symbol R ausgewählt ist aus
1) 5,6-Dioxo-1,4,5,6-tetrahydro-1,2,4-triazin-3-yl, substituiert in 4-Stellung durch
a) einen Alkylrest mit 1 oder 2 Kohlenstoffatomen, gegebenenfalls substituiert durch einen Alkyloxy-, Alkylthio-, Formylrest,
b) einen Allyl-, 2,3-Dihydroxypropyl-, 1,3-Dihydroxy-2-propyl- oder 2-Formyl-2-hydroxyäthyl- rest,
c) einen Alkylrest mit 2 oder 3 Kohlenstoffatomen, substituiert durch Hydroxy, Carbamoyloxy, Alkanoyloxy oder Alkanoylamino (deren Alkanoylteile gegebenenfalls durch Amino substituiert sind), Alkylsulfonylamino, Ureido, Alkylureido oder Dialkylureido,
2) 5,6-Dioxo-1,4,5,6-tetrahydro-1,2,4-triazin-3-yl, substituiert in 1-Stellung durch einen Alkylrest mit 1 oder 2 Kohlenstoffatomen, gegebenenfalls substituiert durch einen Formylrest,
3) 1,3,4-Thiadiazol-5-yl, substituiert durch Alkyl, Dialkylaminoalkyl oder Alkanoylaminoalkyl,
das Symbol R' einen Rest der allgemeinen Formel 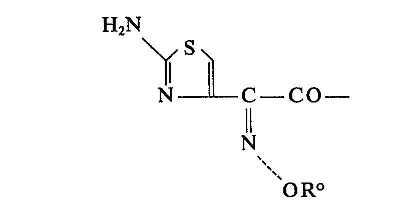 darstellt [worin R° ein Wasserstoffatom, ein Alkyl- oder Vinylrest oder ein Carboxyalkylrest der allgemeinen Formel: 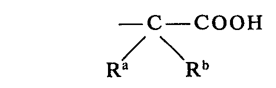 ist, worin die Reste R^{a} und R^{b}, die gleich oder voneinander verschieden sind, Wasserstoffatome oder Alkylreste darstellen oder gemeinsam einen Alkylenrest mit 2 oder 3 Kohlenstoffatomen bilden], oder R' einen a-Carboxyarylacetylrest darstellt, worin Aryl ein Phenylrest (gegebenenfalls substituiert durch einen p-Hydroxyrest) oder ein 2- oder 3-Thienylrest ist, und
das Symbol R" ein Wasserstoffatom oder einen Methoxyrest in Position 7a darstellt, wobei die oben erwähnten Alkyl- oder Alkanoylreste und -teile (ohne besonderen Hinweis) selbstverständlich geradkettig oder verzweigt sind und 1 bis 4 Kohlenstoffatome enthalten,
in ihren E- oder Z-Formen und deren Mischungen,
gegebenenfalls in ihren syn- oder anti-Formen oder deren Mischungen sowie ihrer Metallsalze, ihrer Additionssalze mit Stickstoffbasen und, falls existent, ihrer Säureadditionssalze,
dadurch gekennzeichnet, daß man eine Säure der allgemeinen Formel 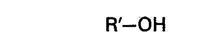 worin R' die obige Bedeutung hat (selbstverständlich sind zutreffendenfalls die in diesem Rest enthaltenen Amin- und Oximfunktionen geschützt) oder ein reaktionsfähiges Derivat dieser Säure auf ein 7-Amino-oxacephalosporin der allgemeinen Formel: 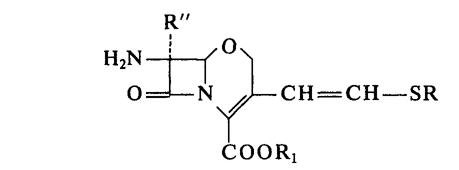 worin R" und R die obige Bedeutung haben und R, ein Wasserstoffatom oder eine Schutzgruppe darstellt, einwirken läßt, dann die Schutzgruppen entfernt und gegebenenfalls die erhaltene Verbindung in ein Metallsalz, ein Additionssalz mit einer Stickstoffbase oder ein Säureadditionssalz überführt, oder daß man ein Thiol (frei oder im Zustand des Alkali- oder Erdalkalisalzes) der allgemeinen Formel 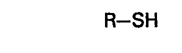 {worin der Substituent von R, der die obige Bedeutung hat, im Acetalzustand geschützt ist [ausgedrückt durch die allgemeinen Formeln 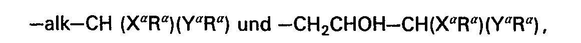 worin alk ein Alkylenrest mit 1 bis 4 Kohlenstoffatomen ist, X" und Y" gleich sind und Sauerstoff- oder Schwefelatome darstellen und R" einen Alkylrest bedeutet oder X" und Y" gleich oder voneinander verschieden sind und Sauerstoff- oder Schwefelatome darstellen und die Reste R" gemeinsam einen Alkylenrest mit 2 oder 3 Kohlenstoffatomen bilden], wenn man eine Verbindung erhalten will, worin R einen Formylrest enthält) auf ein Oxacephalosporinderivat (oder gegebenenfalls eine Isomerenmischung) der allgemeinen Formel: 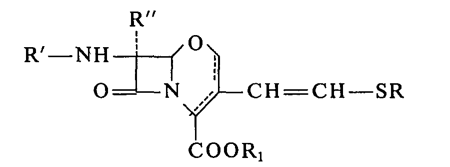 das in 2- oder 3-Bicycloocten-Form vorliegt und worin R₁, R' und R" die obige Bedeutung haben, der Substituent am in 3-Stellung des Bicyclooctens befindlichen Kohlenstoffatom E- oder Z-Stereoisomerie aufweist und R₂ einen Rest der allgemeinen Formeln: 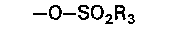 oder 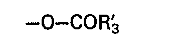 worin R₃ ein geradkettiger oder verzweigter Alkylrest mit 1 bis 4 Kohlenstoffatomen, ein Trifluormethyl-, Trichlormethyl- oder gegebenenfalls durch ein Halogenatom oder einen Alkyl- oder Nitrorest substituierter Phenylrest ist und 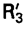 die Bedeutung von R₃ hat oder einen Alkanoylmethyl-, 2-Alkanoyläthyl-, 2-Alkanoylpropyl-, Alkyloxycarbonylmethyl-, 2-Alkyloxycarbonyläthyl- oder 2-Alkyloxycarbonylpropylrest darstellt] oder ein Halogenatom darstellt, einwirken läßt, dann gegebenenfalls vorhandene Schutzgruppen entfernt und gegebenenfalls die erhaltene Verbindung in ein Metallsalz, ein Additionssalz mit einer Stickstoffbase oder ein Säureadditionssalz überführt.

## Claims (Claims for the following Contracting State(s) : BE CH DE FR GB IT LI LU NL SE)

1. An oxacephalosporin of the general formula: 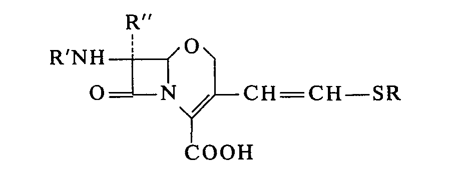 in which the symbol R is chosen from
1) 5,6-dioxo-1,4,5,6-tetrahydro-1,2,4-triazin-3-yl substituted in the 4-position by a) an alkyl radical which contains 1 or 2 carbon atoms and is optionally substituted by an alkoxy, alkylthio or formyl radical, b) an allyl, 2,3-dihydroxypropyl, 1,3-dihydroxyprop-2-yl or 2-formyl-2-hydroxyethyl radical or c) an alkyl radical which contains 2 or 3 carbon atoms and is substituted by hydroxyl, carbamyloxy alkanoyloxy or alkanoylamino (in which the alkanoyl portions are unsubstituted or substituted by amino), alkylsulphonylamino, ureido, alkylureido or dialkylureido,
2) 5,6-dioxo-1,4,5,6-tetrahydro-1,2,4-triazin-3-yl which is substituted in the 1-position by an alkyl radical which contains 1 or 2 carbon atoms and is optionally substituted by a formyl radical, and
3) 1,3,4-thiadiazol-5-yl which is substituted by alkyl, dialkylaminoalkyl or alkanoylaminoalkyl, and the symbol R' represents a radical of the general formula: 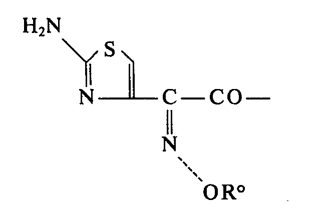 [in which R° is a hydrogen atom, an alkyl, vinyl or carboxyalkyl radical represented by the general formula: 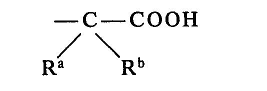 in which the radicals R" and R^{b}, which are identical or different, represent hydrogen atoms or alkyl radicals or together form an alkylene radical containing 2 or 3 carbon atoms], or R' represents an a-carboxyarylacetyl radical in which aryl is a phenyl radical (optionally substituted by a p-hydroxy radical) or a thien-2-yl or thien-3-yl radical, and the symbol R" represents a hydrogen atom or a methoxy radical in the 7a-position, it being understood that the abovementioned alkyl or alkanoyl radicals and portions (unless mentioned specifically) are straight-chain or branched and contain 1 to 4 carbon atoms, in the E or Z forms and mixtures thereof, where appropriate in the syn or anti forms or as mixtures thereof, and its metal salts, addition salts with nitrogen bases and, where they exist, addition salts with acids.

2. A product according to Claim 1, characterised in that the symbol R is a 5,6-dioxo-4-formylalkyl-1,4,5,6-tetrahydro-1,2,4-triazin-3-yl or 2-alkyl-1,3,4-thiadiazol-5-yl radical, the symbol R' is a radical of the general formula (I in which R^{O} is an alkyl radical, and the symbol R" is a hydrogen atom, in the syn or anti forms and E or Z forms or as mixtures thereof, and its metal salts, addition salts with nitrogen bases and addition salts with acids.

3. The E form of the syn isomer of 7-[2-(2-aminothiazol-4-yl)-2-methoxyiminoacetamido]-2-carboxy-3-[2-(2-methyl-1,3,4-thiadiazol-5-yl)thiovinyl]-8-oxo-5-oxa-1-azabicyclo[4.2.0]oct-2-ene.

4. The E form of the syn isomer of 7-[2-(2-aminothiazol-4-yl)-2-methoxyiminoacetamido]-2-carboxy-3-{ 2-[5,6-dioxo-4-(2-oxoethyl)-1,4,5,6-tetrahydro-1,2,4-triazin-3-yl]thiovinyl}-8-oxo-5-oxa-1-azabicyclo[4.2.0]-oct-2-ene.

5. Process for the preparation of a product according to Claim 1, characterised in that an acid of the general formula: 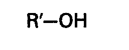 in which R' is defined as in Claim 1 (it being understood that, where relevant, the amine function and the oxime contained in this radical are protected), or a reactive derivative of this acid, is reacted with a 7-aminooxacephalosporin of the general formula: 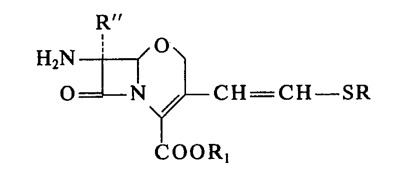 in which R" and R are defined as in Claim 1 and R₁ represents a hydrogen atom or a protective radical, the protective radicals are then removed and, if appropriate, the product obtained is converted into a metal salt, an addition salt with a nitrogen base or an addition salt with an acid.

6. Process according to Claim 5, characterised in that an acid R'OH in which R' is a radical of the general formula: 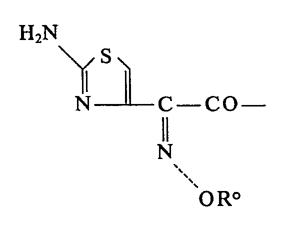 in which R^{o} is defined according to Claim 1, and of which the amine function has first been protected by a tert.-butoxycarbonyl, 2,2,2-trichloroethoxycarbonyl, trichloroacetyl, chloroacetyl, trityl, dibenzyl, benzyl, benzyloxycarbonyl, p-nitrobenzyloxycarbonyl, p-methoxybenzyloxycarbonyl, formyl or trifluoroacetyl grouping, is used.

7. Process according to Claim 5, characterised in that an acid R'OH in which R' is a radical of the general formula: 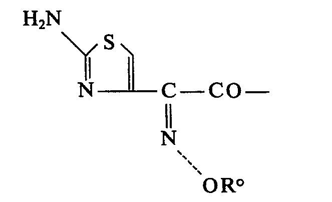 in which R° is a trityl, tetrahydropyrannyl or 2-methoxyprop-2-yl protective group on the oxime function is used.

8. Process according to Claim 5, characterised in that an acid R'OH in which R' is a radical of the general formula: 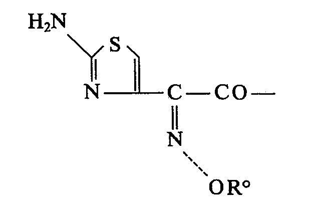 in which R° is a carboxyalkyl grouping (represented by the general formula: 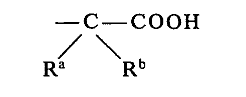 in which R⁸ and R are defined as in Claim 1) which has first been protected by a radical chosen from methoxymethyl, tert.-butyl, benzydryl, benzyl, nitrobenzyl and p-methoxybenzyl, is used.

9. Process according to Claim 5, characterised in that a 7-aminooxacephalosporin in which the easily removable radical represented by R₁ is chosen from the methoxymethyl, tert.-butyl, benzhydryl, benzyl, nitrobenzyl and p-methoxybenzyl radicals, is used.

10. Process according to Claim 5, characterised in that a reactive derivative of the acid of the general formula: 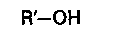 chosen from the anhydride, a mixed anhydride, a reactive ester and a halide of this acid, is used.

11. Process for the preparation of a product according to Claim 1, characterised in that a thiol (free or in the form of an alkali metal or alkaline earth metal salt) of the general formula: 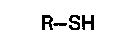 I in which the substituent R, which is defined as in Claim 1, is protected in the acetal state [defined by the general formulae 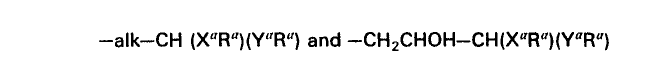 in which alk is an alkylene radical containing 1 to 4 carbon atoms, X" and Y" are identical and represent oxygen or sulphur atoms and R^{α} represents an alkyl radical, or X^{α} and Y^{α} are identical or different and represent oxygen or sulphur atoms and the radicals R^{α} together form an alkylene radical containing 2 or 3 carbon atoms], if the intention is to obtain a product according to Claim 1 in which R contains a formyl radical}, is reacted with an oxacephalosporin derivative (or, where appropriate, with a mixture of isomers) of the general formula: 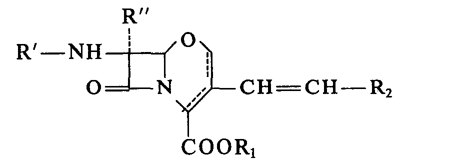 which is in the bicyclooct-2-ene or -3-ene form and in which R' and R" are defined according to Claim 1 and R₁ is defined according to Claim 5, the substituent on the carbon atom in the 3-position of the bicyclooctene gives the E or Z stereoisomerism and R₂ represents a radical of the general formula: 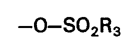 or 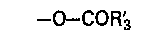 in which R₃ is a straight-chain or branched alkyl radical containing 1 to 4 carbon atoms, trifluoromethyl, trichloromethyl or phenyl which is unsubstituted or substituted by a halogen atom or by an alkyl or nitro radical, and 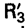 is defined as R₃ or represents an alkanoylmethyl, 2-alkanoylethyl, 2-alkanoylpropyl, alkoxycarbonylmethyl, 2-alkoxycarbonylethyl or 2-alkoxycarbonylpropyl radical], or a halogen atom, and the protective radicals, if these are present, are removed and, if appropriate, the product obtained is converted into a metal salt, an addition salt with a nitrogen base or an addition salt with an acid.

12. Process according to Claim 11, characterised in that the reaction is carried out in the presence of a pyridine or an organic tertiary base.

13. Pharmaceutical composition, characterised in that it contains at least one product according to Claim 1, together with one or more compatible and pharmaceutically acceptable diluents or adjuvants.

## Claims (Claims for the following Contracting State(s) : AT)

Process for the preparation of an oxacephalosporin of the general formula: 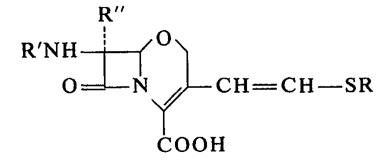 in which the symbol R is chosen from
1) 5,6-dioxo-1,4,5,6-tetrahydro-1,2,4-triazin-3-yl substituted in the 4-position by a) an alkyl radical which contains 1 or 2 carbon atoms and is optionally substituted by an alkoxy, alkylthio or formyl radical, b) an allyl, 2,3-dihydroxypropyl, 1,3-dihydroxyprop-2-yl or 2-formyl-2-hydroxyethyl radical or c) an alkyl radical which contains 2 or 3 carbon atoms and is substituted by hydroxyl, carbamyloxy alkanoyloxy or alkanoylamino (in which the alkanoyl portions are unsubstituted or substituted by amino), alkylsulphonylamino, ureido, alkylureido or dialkylureido,
2) 5,6-dioxo-1 ,4,5,6-tetrahydro-1 ,2,4-triazin-3-yl which is substituted in the 1 -position by an alkyl radical which contains 1 or 2 carbon atoms and is optionally substituted by a formyl radical, and
3) 1,3,4-thiadiazol-5-yl which is substituted by alkyl, dialkylaminoalkyl or alkanoylaminoalkyl, and
the symbol R' represents a radical of the general formula: 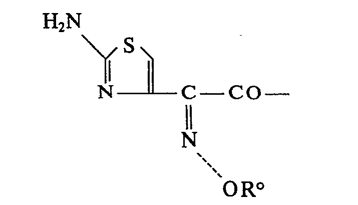 [in which R° is a hydrogen atom, an alkyl, vinyl or carboxyalkyl radical represented by the general formula: 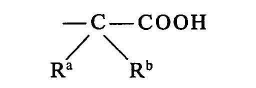 in which the radicals R^{a} and R^{b}, which are identical or different, represent hydrogen atoms or alkyl radicals or together form an alkylene radical containing 2 or 3 carbon atoms], or R' represents an a-carboxyarylacetyl radical in which aryl is a phenyl radical (optionally substituted by a p-hydroxy radical) or a thien-2-yl or thien-3-yl radical, and the symbol R" represents a hydrogen atom or a methoxy radical in the 7a-position, it being understood that the abovementioned alkyl or alkanoyl radicals and portions (unless mentioned specifically) are straight-chain or branched and contain 1 to 4 carbon atoms, in the E or Z forms and mixtures thereof, where appropriate in the syn or anti forms or as mixtures thereof, and its metal salts, addition salts with nitrogen bases and, where they exist, addition salts with acids, characterised in that an acid of the general formula: 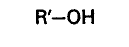 in which R' is defined as above (it being understood that, where relevant, the amine function and the oxime contained in this radical are protected), or a reactive derivative of this acid, is reacted with a 7-aminooxacephalosporin of the general formula: 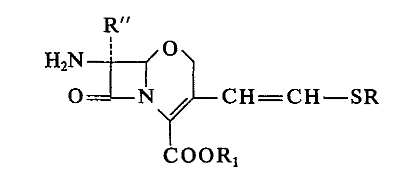 in which R" and R are defined as above and R₁ represents a hydrogen atom or a protective radical, the protective radicals are then removed and, if appropriate, the product obtained is converted into a metal salt, an addition salt with a nitrogen base or an addition salt with an acid, or a thiol (free or in the form of an alkali metal or alkaline earth metal salt) of the general formula: 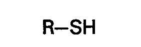 {in which the substituent R, which is defined as above, is protected in the acetal state [defined by the general formulae 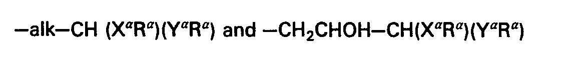 in which alk is an alkylene radical containing 1 to 4 carbon atoms, X" and Y" are identical and represent oxygen or sulphur atoms and R^{α} represents an alkyl radical, or X^{α} and Y^{α} are identical or different and represent oxygen or sulphur atoms and the radicals R^{a}togetherform an alkylene radical containing 2 or 3 carbon atoms], if the intention is to obtain a product in which R contains a formyl radical, is reacted with an oxacephalosporin derivative (or, where appropriate, with a mixture of isomers) of the general formula: 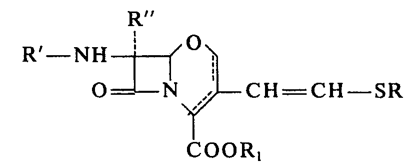 which is in the bicyclooct-2-ene or -3-ene form and in which R₁, R' and R" are defined as above, the substituent on the carbon atom in the 3-position of the bicyclooctene gives the E or Z stereoisomerism and R₂ represents a radical of the general formula: 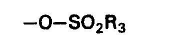 or 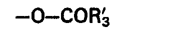 [in which R₃ is a straight-chain or branched alkyl radical containing 1 to 4 carbon atoms, trifluoromethyl, trichloromethyl or phenyl which is unsubstituted or substituted by a halogen atom or by an alkyl or nitro radical, and R3 is defined as R₃ or represents an alkanoylmethyl, 2-alkanoylethyl, 2-alkanoyl- propyl, alkoxycarbonylmethyl, 2-alkoxycarbonylethyl or 2-alkoxycarbonylpropyl radical], or a halogen atom, and the protective radicals, if these are present, are removed and, if appropriate, the product obtained is converted into a metal salt, an addition salt with a nitrogen base or an addition salt with an acid.
